# FASCICULE DE BREVET EUROPEEN

(11) **EP 3 996 729 B1**
(45) Date de publication et mention de la délivrance du brevet: **10.12.2025**
(21) Numéro de dépôt: 20750339.2
(22) Date de dépôt: 09.07.2020
(51) Int. Cl.: A61K 35/747, A61K 36/9066, A61K 31/198, A61P 25/22, A61P 25/24

(54) **COMPOSITION POUR LA PRÉVENTION OU LE TRAITEMENT DE LA DÉPRESSION OU DE L'ANXIÉTÉ**
ZUSAMMENSETZUNG ZUR VORBEUGUNG ODER BEHANDLUNG VON DEPRESSIONEN ODER ANGSTZUSTÄNDEN
COMPOSITION FOR THE PREVENTION OR TREATMENT OF DEPRESSION OR ANXIETY

(30) Priorité: 10.07.2019 FR 1907747
(43) Date de publication de la demande: 18.05.2022
(73) Titulaire: Mousset, Pierre-Yves, 33360 Latresne (FR)
(72) Inventeur: BERACOCHEA, Daniel, BORDEAUX 33800 (FR); LAFAY, Sophie, 91630 MAROLLES EN HUREPOIX (FR); MOUSSET, Pierre-Yves, 33360 LATRESNE (FR)
(74) Mandataire: Aquinov
(86) Numéro de dépôt international: PCT/FR2020/051231
(87) Numéro de publication internationale: WO 2021/005310

(56) Documents cités:
- WO-A1-2011/132831
- WO-A1-2013/004740
- WO-A1-2018/220429
- CN-A- 109 527 273
- JP-A- 2016 199 491
- KR-A- 20090 083 149
- KR-A- 20110 117 591
- ANONYMOUS: "Products: Glutamine Forte Powder - The Way Up", 20 June 2019 (2019-06-20), XP055677691, Retrieved from the Internet <URL:https://web.archive.org/web/20190620231717/http://www.thewayup.com/products/0178.cfm> [retrieved on 20200319]
- KULKARNI S K ET AL: "An Overview of Curcumin in Neurological Disorders", INDIAN JOURNAL OF PHARMACEUTICAL SCIENCES, MEDKNOW PUBLICATIONS PVT LTD, IN, vol. 72, no. 2, 1 March 2010 (2010-03-01), pages 149 - 154, XP002725788, ISSN: 0250-474X, DOI: 10.4103/0250-474X.65012
- NG QIN XIANG ET AL: "Clinical Use of Curcumin in Depression: A Meta-Analysis", JOURNAL OF THE AMERICAN MEDICAL DIRECTORS ASSOCIATION, ELSEVIER, AMSTERDAM, NL, vol. 18, no. 6, 22 February 2017 (2017-02-22), pages 503 - 508, XP085035588, ISSN: 1525-8610, DOI: 10.1016/J.JAMDA.2016.12.071
- ANONYMOUS: "Optimized Superfood Meal Replacement Record ID 10237526", GNPD, 1 October 2005 (2005-10-01), pages 1 - 4, XP055224685, Retrieved from the Internet <URL:http://www.gnpd.com/sinatra/recordpage/10237526/from_search/Ghs8iIeYGW/> [retrieved on 20151030]

## Description

### DOMAINE TECHNIQUE

La présente invention se rapporte au domaine de la santé et concerne plus particulièrement une composition pour la prévention et/ou le traitement des troubles anxieux et/ou des troubles de l'humeur, en particulier de la dépression.

### ARRIERE PLAN TECHNOLOGIQUE

Les troubles psychiatriques sont considérés comme le principal défi sanitaire du 21e siècle, en raison de leur prévalence élevée, 38 % de la population en Europe, et de l'énorme handicap qui y est associé. Les troubles dépressifs caractérisés (TDC) sont les plus fréquents, avec une prévalence de 16 % au cours de la vie. Ces troubles chroniques récidivants ont des conséquences psychosociales majeures et des coûts considérables pour la communauté. Les projections indiquent que les TDC seront le second contributeur aux coûts de prise en charge en 2020, avant d'occuper la 1ère place en 2030.

Depuis des décennies, la principale théorie de la physiopathologie de la dépression est qu'elle résulte de la sous-activité des neuromédiateurs monoamines, en particulier la sérotonine et la noradrénaline. Actuellement, plus de deux tiers des patients traités par un antidépresseur de la classe des inhibiteurs sélectifs de la recapture de la sérotonine (ISRS) prescrit en 1ère intention ne présentent pas de rémission. De plus, 20 à 30% des patients résistent à l'ensemble des stratégies médicamenteuses. Une composition comprenant de la glutamine et du curcumin a été précédemment décrite (https://web.archive.org/web/20190620231717/http://www.thewayup.com/products/0178.cfm).

Au cours de la dernière décennie, la compréhension de la physiopathologie de la dépression a beaucoup évolué avec la contribution de nouvelles technologies. Ainsi, l'imagerie cérébrale a montré que la dépression est associée à une perte de 'matière blanche' intéressant notamment le cortex préfrontal médian qui intervient dans la régulation des émotions. Or, les astrocytes qui y résident sont des acteursclé de la régulation de neuromédiateurs qui n'appartiennent pas à la famille des monoamines mais au cycle glutamine-glutamate-GABA.

Par ailleurs, de nombreux travaux montrent également d'importantes altérations biologiques associées à la dépression, notamment un syndrome immuno-inflammatoire, des altérations de l'axe corticotrope, principal axe neuroendocrinien de réponse au stress, des anomalies métaboliques, et un stress oxydatif. Des données récentes, provenant principalement d'études animales, indiquent que ces voies, mal régulées dans la dépression, sont modulées par le microbiote intestinal.

Décrit comme « un organe additionnel » et composé de plus de 1000 espèces bactériennes différentes répertoriées, le microbiote intestinal peut également jouer un rôle bénéfique pour l'hôte en exerçant de nombreuses fonctions biologiques, telles que l'aide à la digestion, à l'absorption des nutriments et à la métabolisation des substrats, la lutte contre les pathogènes, le maintien de l'intégrité de l'épithélium intestinal ou encore la mise en place et l'homéostasie de réponses immunitaires.

Des études utilisant des approches différentes mais complémentaires, telles que des rongeurs nés et élevés sans microbes (Germfree), des antibiotiques, des probiotiques, des travaux sur les infections gastro-intestinales et sur la transplantation de microbiote fécal, ont montré que le microbiote intestinal contribue également à la régulation du système nerveux central. Cette interaction est qualifiée d' « axe intestin-cerveau » défini comme un système de communication bidirectionnel entre le système nerveux central et l'appareil digestif par l'intermédiaire de voies neuronales, neuroimmunes et neuroendocrines notamment. Du point de vue clinique, des symptômes digestifs sont très fréquemment associés aux troubles psychiatriques. Ainsi, 60 à 85% des patients souffrant d'un syndrome du côlon irritable ont des symptômes psychiatriques, dont 20 à 40% présentent une dépression caractérisée, généralement plus sévère que pour la population normale.

La découverte des mécanismes par lesquels les bactéries commensales sont impliquées dans le fonctionnement du cerveau ouvre ainsi la voie au développement de nouvelles perspectives thérapeutiques basées sur le microbiote pour la prise en charge des troubles anxiodépressifs.

Malgré les progrès réalisés dans la connaissance des troubles anxio-dépressifs, le besoin d'alternatives aux traitements médicamenteux actuellement disponibles demeure majeur.

### RESUME DE L'INVENTION

Les inventeurs ont montré que, de manière tout à fait surprenante, il existe une synergie d'action entre la glutamine, un extrait de curcuma et *Lactobacillus rhamnosus* pour la régulation des symptômes anxiodépressifs, résultant ainsi en une amélioration comparable à celle obtenue avec un médicament antidépresseur de référence dans ce domaine (la clomipramine). Cette combinaison diminue significativement les troubles de l'anxiété et de la dépression.

L'invention est exposée dans l'ensemble de revendications annexé.

L'invention concerne une composition pharmaceutique, nutraceutique ou alimentaire comprenant de la glutamine, un extrait de curcuma et du *Lactobacillus rhamnosus* dans les proportions suivantes :
- de 90 à 96% en poids de glutamine,
- de 3 à 6% en poids d'extrait de curcuma,
- de 0,25% à 5% en poids de *Lactobacillus rhamnosus,*
pour 100% en poids de la somme de glutamine, d'extrait de curcuma et de *Lactobacillus rhamnosus,* et dans laquelle l'extrait de curcuma comprend au moins 15% en poids de curcuminoïdes et est associé à une cyclodextrine.

De préférence, la composition comprend de 90 à 96% en poids de glutamine, de 3 à 6% en poids d'extrait de curcuma, et de 0,25 à 5% en poids de *Lactobacillus rhamnosus,* pour 100% en poids de la composition pharmaceutique, nutraceutique ou alimentaire.

Dans un aspect particulier, la composition comprend de 85 à 95% en poids de glutamine, de 3 à 10% en poids d'extrait de curcuma, et de 0,25 à 5% en poids de *Lactobacillus rhamnosus,* pour 100% en poids de la somme de glutamine, d'extrait de curcuma et *Lactobacillus rhamnosus.*

Dans un aspect particulier, la cyclodextrine est une gamma-cyclodextrine.

En particulier, la souche de *Lactobacillus rhamnosus* utilisée dans la composition est la souche *Lactobacillus rhamnosus GG.*

De préférence, les bactéries *Lactobacillus rhamnosus* sont sous forme vivante.

La composition selon l'invention peut comprendre en outre : un ou plusieurs prébiotiques ; et/ou des vitamines, minéraux et/ou oligo-élements ; et/ou des poudres ou extraits de plantes, fruits, légumes, algues ou champignons ; et/ou un ou plusieurs microorganismes probiotiques choisis dans le groupe constitué par des bactéries du genre Bacillus, Bacteroides, Enterobacteriaceae, Enterococcus, Faecalibacterium, Fusobacterium, Lactobacillus, Odoribacter, Parabacteroides, Pediococcus, Roseburia, Ruminococcus, et Streptococcus et/ou des levures, en particulier du genre Saccharomyces.

L'invention concerne également une composition selon l'invention pour son utilisation en tant que complément alimentaire ou médicament, en particulier à usage humain ou vétérinaire. L'invention concerne également une composition selon l'invention pour son utilisation dans la prévention ou le traitement de la dépression ou l'anxiété, la composition selon l'invention étant administrée par voie orale. Selon un mode de réalisation, la composition est sous une forme destinée à être administrée de telle sorte que la dose journalière de glutamine est comprise entre 0,5 et 10 g, la dose journalière d'extrait de curcuma est comprise entre 25 mg et 500 mg et la dose journalière de *Lactobacillus rhamnosus* est comprise entre 0,05 mg et 500 mg ou entre 1x10⁷ CFU et 1x10¹¹ CFU.

Dans un aspect particulier, ladite composition se présentant sous la forme d'unités de dose, par exemple sous la forme de comprimés ou de gélules, chaque unité de dose comprenant :
- de 0,5 g à 10 g de glutamine,
- de 25 mg à 500 mg d'extrait de curcuma, et
- de 0,05 mg à 500 mg ou entre 1x10⁷ CFU et 1x10¹¹ CFU de *Lactobacillus rhamnosus.*

Dans un autre aspect particulier, chaque unité de dose comprend :
- de 0,5 g à 2 g de glutamine,
- de 50 mg à 100 mg d'extrait de curcuma, et
- de 10 mg à 20 mg de *Lactobacillus rhamnosus.*

L'invention a finalement trait à un kit pharmaceutique, nutraceutique ou alimentaire comprenant la composition selon l'invention, dans lequel la composition est sous la forme de compositions distinctes, comprenant :
- une première composition comprenant la bactérie *Lactobacillus rhamnosus,* par exemple associée à un ou plusieurs excipients, et
- une seconde composition comprenant la glutamine et l'extrait de curcuma, par exemple associée à un ou plusieurs excipients.

L'invention a finalement trait à un kit pharmaceutique, nutraceutique ou alimentaire selon l'invention pour son utilisation en tant que complément alimentaire ou médicament, en particulier à usage humain ou vétérinaire, ou pour son utilisation dans la prévention ou le traitement de la dépression ou l'anxiété.

### DESCRIPTION DES DESSINS

**Fig. 1** représente le déroulement de l'étude *in vivo.*
**Fig. 2** représente le pourcentage du temps dans les bras ouvert lors du test « labyrinthe en croix surélevé (EPM)». Les résultats sont les moyennes +/- l'erreur-type. *** p<0,001 vs « contrôle » et « contrôle + placebo ».
**Fig. 3** représente le pourcentage d'immobilité lors du test de suspension par la queue. Les résultats sont les moyennes +/- l'erreur-type. *** p<0,001 vs « contrôle » et « contrôle + placebo».
**Fig. 4** représente les performances du test en espace ouvert (open-field) de souris stressées âgées de 6 mois après 3 semaines de traitement comparé à des souris stressées et traitées avec placebo ou des souris non stressées. Les résultats sont les moyennes +/- l'erreur-type. * représente une différence significative en comparaison du groupe Modèle / Placebo : * p < 0,05 ; ** p < 0,01 ; *** p < 0,001 ; # représente une différence significative en comparaison du groupe Contrôle / Placebo : # : p<0,05 et ## : p<0,01

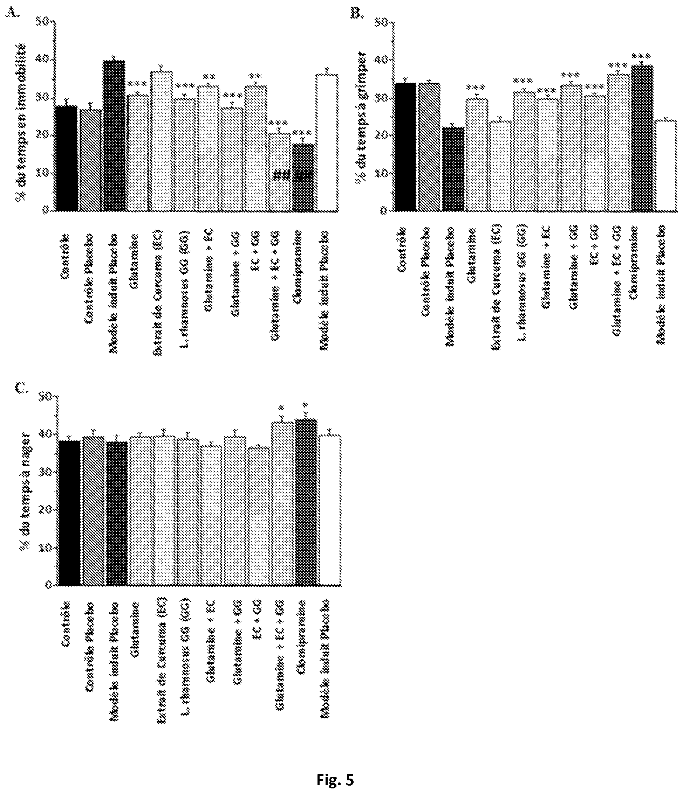

**Fig. 5** représente les performances au test de nage forcée de souris stressées âgées de 6 mois après 3 semaines de traitement comparé à des souris stressées et traitées avec placebo ou des souris non stressées. A - Pourcentage du temps d'immobilité ; B - Pourcentage du temps d'escalade ; C - Pourcentage du temps de nage. Les résultats sont les moyennes +/- l'erreur-type. * représente une différence significative en comparaison du groupe Modèle / placebo : * p < 0,05; ** p < 0,01; *** p < 0,001 ; # représente une différence significative en comparaison du groupe Contrôle / Placebo : # : p<0,05 et ## : p<0,01.
**Fig. 6** représente la diversité microbienne. PS = Placebo group / Start (before treatment); PE= Placebo group / End (after treatment); FFS = Full formulation / Start; FFE = Full formulation / End ; CS= Clomipramine group / Start; CE= Clomipramine group / End
**Fig. 7** représente la composition du microbiote intestinal (répartition en pourcentage par famille). PS = Placebo group / Start (before treatment); PE= Placebo group / End (after treatment); FFS = Full formulation / Start; FFE = Full formulation / End ; CS= Clomipramine group / Start; CE= Clomipramine group / End.
**Fig. 8** représente A - La diversité béta (β) du microbiote intestinal évaluée par l'analyse en composantes principales (PCoA). Le PCoA a été calculé aux niveaux des genres à l'aide de la matrice de dissimilarité de Bray-Curtis. Les deux premières composantes sont affichées et résument 57% de l'inertie des données. B - Diagramme à bandes illustrant les distances entre les échantillons et le profil du microbiote centroïde de chaque groupe de souris.

### DESCRIPTION DETAILLEE DE L'INVENTION

### Introduction

L'invention concerne une composition comprenant un extrait de curcuma, de la glutamine et une souche bactérienne *Lactobacillus rhamnosus,* dans laquelle la composition comprend des proportions particulières de ces trois ingrédients, de préférence de 90 à 96% en poids de glutamine, de 3 à 6% en poids d'extrait de curcuma, et de 0,25 à 5% en poids de *Lactobacillus rhamnosus,* pour 100% de la composition. Cette composition est notamment une composition pharmaceutique, par exemple pour la préparation de médicaments, ou une composition nutraceutique, par exemple pour la préparation de compléments alimentaires, ou une composition alimentaire pour la préparation d'aliments, aliments fonctionnels ou d'aliments pour groupes spécifiques.

L'invention concerne également l'utilisation de cette composition pour prévenir ou traiter les troubles anxieux et la dépression, chez un sujet.

Les inventeurs ont en effet montré que de manière surprenante, il existait une synergie d'action entre ces trois ingrédients en termes de régulation des symptômes anxio-dépressifs, résultant en une amélioration du bien-être mental bien supérieure à l'effet attendu.

Les inventeurs ont particulièrement montré :
- que l'association de la glutamine et de *Lactobacillus rhamnosus* permettait de diminuer les troubles de l'anxiété et de la dépression de façon équivalente à la glutamine ou au lactobacille seul ;
- que l'association de la glutamine ou du lactobacille avec un extrait de curcuma tendait à diminuer les effets de la glutamine ou du lactobacille seul ;
- que l'association de la glutamine, de *Lactobacillus rhamnosus* et d'un extrait de curcuma permettait de diminuer de façon très significative les troubles de l'anxiété et de la dépression comparée aux ingrédients testés indépendamment ou aux ingrédients mélangés deux à deux ;
- que l'association de la glutamine, à la bactérie *Lactobacillus rhamnosus* et à un extrait de curcuma permettait de façon surprenante d'obtenir des résultats comparables à ceux obtenus avec un médicament de référence pour le traitement de la dépression (clomipramine).

Les inventeurs décrivent ainsi pour la première fois l'intérêt d'une composition à base de glutamine, d'extrait de curcuma et de *Lactobacillus rhamnosus* dans le domaine de la psychiatrie, ainsi que sa capacité à réguler l'humeur ou l'état émotionnel d'un sujet, notamment en réduisant l'anxiété et/ou la dépression chez un sujet, ainsi que les symptômes y étant associés. Cette composition peut ainsi être avantageusement administrée à des sujets présentant ou étant à risque de développer des états anxieux et/ou dépressifs, en particulier des symptômes anxio-dépressifs.

### Définitions

Tel qu'utilisé ici, les termes « désordre », « anomalie » ou « trouble » sont interchangeables et font référence à un organe, une partie, une structure ou un système du corps qui fonctionne incorrectement. De préférence, le terme « trouble » désigne un trouble de la santé, par exemple perturbant les fonctions physiques ou mentales normales. De préférence, le trouble est un trouble anxieux et/ou un trouble de l'humeur, en particulier la dépression, ou une symptomatologie anxieuse et/ou un épisode dépressif isolés.

Tel qu'utilisé ici, les termes « troubles anxieux », « l'anxiété » et « symptôme anxiogène » sont interchangeables et représentent une catégorie de troubles mentaux caractérisés par des sentiments négatifs, en particulier une inquiétude et/ou une nervosité face aux événements à venir et/ou une peur et/ou une nervosité en réaction aux événements en cours. L'anxiété peut se caractériser par un état émotionnel pénible caractérisé par une peur "sans objet", un sentiment d'attente inquiète, et des manifestations neuro-végétatives souvent au premier plan telles que des palpitations, une oppression thoracique, une gêne respiratoire, une "boule dans la gorge", etc... Les troubles anxieux incluent, sans toutefois s'y limiter, les troubles anxieux généralisés, la phobie, la panique, l'hypochondrie, les troubles obsessionnels compulsifs, le stress, le stress post-traumatique, l'anxiété sociale, l'anxiété de séparation, l'anxiété de fin de vie et l'anxiété situationnelle.

Les termes « trouble de l'humeur », « troubles thymiques » et « troubles affectifs » font référence ici à une condition caractérisée par une perturbation de la régulation émotionnelle ou comportementale d'un sujet, qui reflète un dysfonctionnement des processus psychologiques, biologiques et/ou du développement sous-jacents à la fonction mentale. En particulier, ces termes font référence à une altération négative de l'humeur ou de l'état émotionnel d'un sujet. Les troubles de l'humeur incluent, sans toutefois s'y limiter, les troubles dépressifs, les troubles bipolaires, la dysthymie, le trouble affectif saisonnier (TAS) ou un trouble mixte anxio-dépressif.

Les « trouble dépressifs », les «symptômes dépressifs» ou la « dépression » sont entendus ici comme une altération négative de l'humeur d'un sujet, en particulier une humeur basse, un manque d'intérêt, un ralentissement ou une agitation psychomoteur, des changements d'appétit, une concentration insuffisante ou une indécision, ou d'autres symptômes cognitifs associés à la dépression, tels qu'une culpabilité excessive ou des sentiments d'inutilité, de manque d'énergie ou de fatigue, conduisant éventuellement à des idées suicidaires. Un « symptôme dépressif » comprend ainsi tout sentiment de tristesse et de perte d'intérêt, et est typiquement basé sur un déséquilibre d'un ou plusieurs neurotransmetteurs. Les troubles dépressifs incluent, sans toutefois s'y limiter, la dépression, la dépression majeure, la dépression mélancolique, la dépression post-partum, la dépression atypique.

Tel qu'utilisé ici, le « bien-être » désigne un état positif de santé ou de confort, par exemple par rapport à une population de référence ou à un état précédent. Tel qu'utilisé ici, le « bien-être mental » se réfère à un état mental positif, par rapport à une population de référence ou un état émotionnel précédent. Par exemple, chez un individu dépressif, une faible estime de soi ou une anxiété peuvent connaître une amélioration du bien-être mental en réponse à un traitement visant à améliorer l'humeur, l'estime de soi ou l'anxiété. Le « bien être mental » peut s'accompagner d'un « bien-être physique » qui fait référence à un ou plusieurs aspects positifs de la santé physique d'un individu et comprend par exemple le soulagement des symptômes somatiques associés à un trouble de l'humeur, en particulier à une dépression ou une anxiété.

Le terme « traitement » se rapporte ici à l'obtention d'un effet pharmacologique, mental et/ou physiologique souhaité. L'effet peut être prophylactique en termes de prévention totale ou partielle d'un trouble ou d'un symptôme et/ou peut être thérapeutique en termes de guérison partielle ou complète d'un trouble et/ou d'un effet indésirable attribuable celui-ci. Le terme « traitement » tel qu'utilisé ici couvre tout traitement d'une maladie ou d'un trouble chez un mammifère, en particulier chez un humain, pour : réduire l'incidence et/ou le risque de rechute de la maladie ou du trouble pendant une période sans symptômes; soulager ou réduire un symptôme de la maladie ou du trouble; empêcher la maladie ou le trouble de se produire ou de se reproduire chez un sujet qui peut être prédisposé à la maladie mais qui n'a pas encore été diagnostiqué comme l'ayant; inhiber la maladie ou le trouble, c'est-à-dire stopper son développement (par exemple, réduire le taux de progression); réduire la fréquence des épisodes de la maladie ou du trouble; et soulager la maladie ou le trouble, c'est-à-dire provoquer une régression totale ou partielle de la maladie ou du trouble.

Les termes « individu », « hôte », « sujet » et « patient », sont utilisés ici de manière interchangeable, et désignent un mammifère, plus particulièrement un sujet animal et plus particulièrement encore un humain. Lorsqu'il s'agit d'un animal, l'animal peut être un animal domestique tel qu'un chat ou un chien, ou un animal d'élevage tel qu'un cheval.

Telle qu'utilisée ici, une « composition pharmaceutique » désigne une préparation d'un ou plusieurs des agents actifs avec d'autres composants chimiques optionnels tels que des supports physiologiquement appropriés et/ou des excipients. Le but d'une composition pharmaceutique est de faciliter l'administration de l'agent actif à un organisme. Les compositions de la présente invention peuvent se présenter sous une forme appropriée pour toute voie d'administration ou d'utilisation conventionnelle. La composition pharmaceutique selon l'invention englobe les compositions pharmaceutiques utilisées en médecine humaine et les compositions pharmaceutiques utilisées en médecine animale, c'est à dire les compositions vétérinaires. De préférence, la composition pharmaceutique comprend en outre un véhicule pharmaceutiquement acceptable.

Tel qu'utilisé ici, les termes « support pharmaceutiquement acceptable » ou « excipient pharmaceutiquement acceptable » ou « véhicule pharmaceutiquement acceptable » sont interchangeables et comprennent tous composés ou combinaisons de composés qui sont connus de l'homme de l'art comme étant utiles dans la formulation de compositions pharmaceutiques ou vétérinaires. Dans le contexte de la présente invention, on entend par «physiologiquement acceptable » ou « pharmaceutiquement acceptable », tout milieu ou additif qui n'interfère pas avec l'efficacité de l'activité biologique du principe actif (ici, la combinaison de glutamine, d'extrait de curcuma et de *L. rhamnosus*), et qui n'est pas excessivement toxique pour le sujet, aux concentrations auxquelles il est administré et/ou qui ne produisent pas de réaction indésirable lorsqu'elles sont administrées à un humain ou à un animal. Un véhicule, un support ou un excipient physiologiquement acceptable peut être approprié à l'administration aux humains et/ou aux animaux (en particulier aux mammifères).

Le terme « médicament », tel qu'utilisé ici, englobe des médicaments à usage humain et animal en médecine humaine et vétérinaire et fait référence à toute substance acceptable sur le plan pharmacologique qui procure un effet thérapeutique et/ou bénéfique. Le terme « médicament » utilisé ici n'est pas nécessairement limité aux substances nécessitant une autorisation de mise sur le marché, mais peut comprendre des substances qui peuvent être utilisées dans les cosmétiques et les remèdes naturels.

Le terme « nutraceutique » fait référence à une composition ou produit fabriqué à partir de substances alimentaires, mais rendu disponible sous forme de comprimé, de poudre, de potion ou d'autre formes galéniques habituellement non associées à des aliments, et ayant un effet physiologique bénéfique ou protecteur contre des troubles ou maladies animales ou humaines. On retrouve notamment sous cette définition les compléments alimentaires, certains aliments pour groupe spécifique ou les substituts de repas.

Par « complément alimentaire », est entendu ici toute composition qui est formulée et administrée séparément d'autres aliments et a pour but de complémenter les apports nutritionnels d'un sujet ayant une forme acceptable sur le plan pharmacologique, notamment sous forme de gélules, comprimés, capsules molles, sachets, stick-packs, sirop, compte-gouttes, ou toute autre forme adaptée bien connue de l'homme du métier.

Les termes « aliment », « produit alimentaire » et « denrée alimentaire » sont utilisés de manière interchangeable ici et comprennent, en plus des aliments couramment consommés par les humains et les animaux comme des animaux domestiques ou d'élevage, des aliments fonctionnels et les aliments pour groupes spécifiques, eux même comprenant les denrées destinées à des fins médicales spéciales.

Le terme « aliment fonctionnel » fait référence ici à un aliment conventionnel faisant partie de l'alimentation normale et procurant des bienfaits physiologiques et/ou réduisant le risque de maladies ou de troubles et/ou réduisant les symptômes y étant associés, au-delà des nutriments traditionnels qu'il contient.

Tel qu'utilisé ici, le terme « additif alimentaire » se réfère à une composition qui est destinée à être mélangée avec un ou plusieurs autres aliments avant d'être administrée à un sujet. Plus particulièrement, on entend par «additif alimentaire» tout additif tel que défini par le Codex alimentarius, Norme générale pour les additifs alimentaires - Codex Stan 192-1995, c'est à dire toute substance qui n'est pas consommée seule en tant que denrée alimentaire, ni utilisée seule comme ingrédient caractéristique d'une denrée alimentaire, qu'elle ait ou non une valeur nutritive, et dont l'addition intentionnelle à une denrée alimentaire dans un but technologique (y compris organoleptique) à une étape quelconque de la fabrication, de la transformation, de la préparation, du traitement, du conditionnement, de l'emballage, du transport ou de l'entreposage de ladite denrée entraîne, ou peut, selon toute vraisemblance, entraîner, directement ou indirectement, son incorporation ou celle de ses dérivés dans cette denrée ou en affecter d'une autre façon les caractéristiques.

Tel qu'utilisé ici, un « prébiotique » se réfère à un ingrédient ou substrat qui, utilisé sélectivement par un microorganisme de l'hôte, confère un bénéfice sur la santé. Il peut permettre d'induire des changements bénéfiques, à la fois dans la composition et/ou l'activité d'un probiotique. Un prébiotique peut être un aliment ou une boisson comestible ou un ingrédient de ceux-ci. Les prébiotiques peuvent inclure des hydrates de carbones complexes, des polyphénols et des acides gras polyinsaturés. Un prébiotique est habituellement un glucide non digestible tel qu'un oligo- ou polysaccharide, ou un alcool de sucre, qui n'est pas dégradé ou absorbé dans le tube digestif supérieur en l'absence de micro-organismes intestinaux. De préférence, les prébiotiques selon l'invention sont tels que définis dans l'Arrêté du 26 septembre 2016 établissant la liste des substances à but nutritionnel ou physiologique autorisées dans les compléments alimentaires et les conditions de leur emploi.

Tel qu'utilisé ici, le terme « probiotique » se réfère à des bactéries vivantes ou inactivées qui, lorsqu'elles sont administrées en quantités adéquates, ont un effet bénéfique sur l'organisme hôte. Ces compositions sont avantageuses en ce qu'elles sont appropriées pour une administration aux humains et à d'autres sujets mammifères. Les substances probiotiques contiennent un nombre suffisamment élevé de microorganismes probiotiques pour exercer une action directe ou indirecte sur le microbiote intestinale. Il est à noter que, aux fins de la présente description, on entend par probiotique toute forme biologiquement active de probiotique, de préférence mais non limitativement, les lactobacilles, bifidobactéries, bacillus, streptocoques, entérocoques, propionibactéries ou saccaromycètes mais encore d'autres microorganismes constituant la « flore intestinale normale », ou encore des fragments de la paroi bactérienne ou de l'ADN de ces microorganismes. De préférence, les probiotiques selon l'invention sont tels que définis par l'Organisation des Nations unies pour l'alimentation et l'agriculture (FAO) et l'Organisation Mondiale de la Santé (OMS) dans la Consultation mixte d'experts FAO/OMS sur l'évaluation des propriétés sanitaires et nutritionnelles des probiotiques dans les aliments, y compris le lait en poudre contenant des bactéries lactiques vivantes réalisée en 2001. Dans un aspect particulier, on entend par « bactérie probiotique » des bactéries de l'espèce *Lactobacillus rhamnosus,* plus particulièrement de la souche *Lactobacillus rhamnosus GG.*

Les termes « microbiote » ou « microflore » utilisés ci-après sont interchangeables et désignent l'ensemble des espèces microbiennes présentes (de manière durable ou transitoire) dans un environnement, comprenant des eucaryotes, des archées, des procaryotes et des virus. Le terme « microbiome » fait référence au « contenu génétique » du microbiote et comprend l'ADN génomique, l'ARN ribosomique, l'épigénome, les plasmides et tous les autres types d'informations génétiques du microbiote. Préférentiellement, ces termes font référence ici à des bactéries du microbiote intestinal humain ou animal.

Tel qu'utilisé ici, le terme « bactérie » désigne tout micro-organisme procaryote existant sous la forme d'une cellule unique, dans un groupe ou dans un agrégat de cellules individuelles. Le terme « bactérie » englobe toutes les variantes de bactéries (par exemple, les bactéries endogènes ou les bactéries environnementales), plus particulièrement les bactéries du microbiote humain ou animal, notamment le microbiote intestinal. Les bactéries de la présente invention appartiennent aux subdivisions bactériennes Gram-positives, particulièrement de l'espèce *Lactobacillus rhamnosus,* préférentiellement de la souche GG.

Tel qu'utilisé ici, le terme « comprenant » ou « comprend » est utilisé en référence à des substances, composés ou procédés qui sont essentiels à l'invention, mais qui sont ouverts à l'inclusion d'éléments non spécifiques, essentiels ou non. L'utilisation de « comprenant » indique l'inclusion plutôt que la limitation.

Le terme « et/ou » tel qu'il est utilisé ici doit être considéré comme une description spécifique de chacune des deux caractéristiques ou composants spécifiés, avec ou sans l'autre. Par exemple, «A et/ou B» doit être considéré comme une divulgation spécifique de chacun des éléments suivants : (i) A, (ii) B et (iii) A et B, comme si chacun d'eux était présenté individuellement.

Le terme « environ » tel qu'utilisé ici en relation avec toutes les valeurs (incluant les extrémités inférieures et supérieures de plages numériques) signifie toute valeur ayant une variation acceptable t allant jusqu'à +/- 10% (par exemple, +/- 0,5% , +/- 1%, +/- 1,5%, +/- 2%, +/- 2,5%, +/- 3%, +/- 3,5%, +/- 4%, +/- 4,5% , +/-5%, +/- 5,5%, +/- 6%, +/- 6,5%, +/- 7%, +/- 7,5%, +/- 8%, +/- 8,5%, + / - 9%, +/- 9,5%). L'utilisation du terme "environ" au début d'une liste de valeurs modifie chacune d'entre elles (c'est-à-dire « environ 1, 2 et 3 » se réfère à environ 1, environ 2 et environ 3). En outre, lorsqu'une liste de valeurs est décrite (par exemple environ 50%, 60%, 70%, 80%, 85% ou 86%), la liste inclut toutes ses valeurs intermédiaires et fractionnaires (par exemple 54% ou 85,4%).

Le terme « consiste essentiellement en » tel qu'utilisé ici pour définir une composition dans laquelle les éléments autres que ceux-mentionnés ne représentent pas plus 10% en poids de la composition, de préférence pas plus de 5 % en poids, et de manière plus spécifique pas plus de 1 % en poids.

### Compositions pharmaceutiques, nutraceutiques ou alimentaires

La présente invention a pour objet une composition alimentaire, nutraceutique, pharmaceutique ou vétérinaire comprenant ou consistant essentiellement en de la glutamine, un extrait de curcuma et un probiotique de l'espèce *Lactobacillus rhamnosus,* de préférence en des proportions particulières, notamment entre ces trois ingrédients. La combinaison de ces trois ingrédients constitue le principe actif de la composition.

Selon l'invention, on entend par « combinaison d'ingrédients actifs » ou encore « association d'ingrédients actifs » un ensemble d'actifs pouvant agir de manière coopérative ou synergique afin d'obtenir un ou plusieurs effets pharmacologiques, et/ou physiologiques et/ou nutritionnels visant à améliorer l'état général d'un sujet et/ou traiter ou prévenir une pathologie ou l'un de ses symptômes ou troubles associés. La combinaison d'ingrédients actifs selon l'invention trouve particulièrement une application dans la prise en charge des sujets souffrant ou susceptibles de souffrir d'un ou plusieurs troubles des émotions.

Dans un premier aspect qui n'est pas revendiqué, la composition comprend de la glutamine, un extrait de curcuma et du *Lactobacillus rhamnosus,* de préférence de la souche *Lactobacillus rhamnosus GG,* la glutamine, l'extrait de curcuma et les bactéries *Lactobacillus rhamnosus* étant présents dans la composition dans les proportions suivantes :
- de 50 à 95% en poids de glutamine,
- de 1 à 15% en poids d'extrait de curcuma,
- de 0,02% à 5% de probiotique *Lactobacillus rhamnosus,* de préférence de la souche *Lactobacillus rhamnosus GG,*
pour 100% en poids de la somme de glutamine, d'extrait de curcuma et *Lactobacillus rhamnosus.*

De préférence, l'extrait de curcuma contient au minimum 10%, 15% ou 20% en poids de curcuminoïdes. Dans un mode de réalisation particulier, l'extrait de curcuma est un extrait standardisé de curcuma. Dans un aspect particulier, l'extrait de curcuma est encapsulé et de préférence encore l'extrait de curcuma est encapsulé avec une cyclodextrine, notamment de la gamma-cyclodextrine.

Dans un aspect plus particulier qui n'est pas revendiqué, la composition comprend ou consiste essentiellement en :
- de 50 à 95% en poids de glutamine,
- de 1 à 15% en poids d'extrait de curcuma,
- de 0,02% à 5% en poids de probiotique *Lactobacillus rhamnosus,* de préférence de la souche *Lactobacillus rhamnosus GG,*
pour 100% en poids de la composition.

Dans un autre aspect particulier qui n'est pas revendiqué, la composition peut comprendre ou consister essentiellement en :
- de 50 à 95% en poids de glutamine,
- de 0,1% à 4,5% en poids de curcuminoïdes

- de 0,02% à 5% en poids de probiotique *Lactobacillus rhamnosus,* de préférence de la souche *Lactobacillus rhamnosus GG,*
pour 100% en poids de la somme de glutamine, extrait de curcuma et *Lactobacillus rhamnosus* ou pour 100 % en poids de la composition.

Dans un autre aspect particulier qui n'est pas revendiqué, la composition comprend ou consiste essentiellement en :
- de 50 à 95% en poids de glutamine,
- de 0,1 à 3,3% en poids de curcumine,
- de 0,02% à 5% en poids de probiotique *Lactobacillus rhamnosus,* de préférence de la souche *Lactobacillus rhamnosus GG,*
pour 100% en poids de la composition ou pour 100% en poids de la somme de la glutamine, curcumine et *Lactobacillus rhamnosus.*

Dans un autre aspect particulier qui n'est pas revendiqué, la composition comprend ou consiste essentiellement en :
- de 70% à 97% en poids de glutamine, de préférence de 75, 80, 85, 90% à 95, 96 ou 97% en poids de glutamine,
- de 3% à 10%, de préférence de 3% à 6%, de manière toute préférée de 4% à 5 % en poids d'extrait de curcuma,
- de 0,25% à 5%, de préférence de 0,3% à 1%, préférentiellement de 0,3% à 0,6%, de manière toute préférée de 0,4% à 0,5% en poids de probiotique *Lactobacillus rhamnosus,* la souche étant de préférence *Lactobacillus rhamnosus GG,*
pour 100% en poids de la composition ou pour 100% en poids de la somme de la glutamine, curcumine et *Lactobacillus rhamnosus.*

Dans un autre aspect particulier qui n'est pas revendiqué, la composition comprend ou consiste essentiellement en :
- de 75% à 95% en poids de glutamine, de préférence de 75, 80, 85, 90% à 95, 96 ou 97% en poids de glutamine,
- de 3% à 10%, de préférence de 3% à 6%, de manière toute préférée de 4% à 5 % en poids d'extrait de curcuma,
- de 0,25% à 5%, de préférence de 0,3% à 1%, préférentiellement de 0,3% à 0,6%, de manière toute préférée de 0,4% à 0,5% en poids de probiotique *Lactobacillus rhamnosus,* la souche étant de préférence *Lactobacillus rhamnosus GG,*
pour 100% en poids de la composition ou pour 100% en poids de la somme de la glutamine, curcumine et *Lactobacillus rhamnosus.*

Dans un autre aspect particulier qui n'est pas revendiqué, la composition comprend ou consiste essentiellement en :
- de 85% à 97% en poids de glutamine, de préférence 85% à 95% en poids de glutamine,
- de 3% à 10%, de préférence de 3% à 6%, de manière toute préférée de 4% à 5 % en poids d'extrait de curcuma,
- de 0,25% à 5%, de préférence de 0,3% à 1%, préférentiellement de 0,3% à 0,6%, de manière toute préférée de 0,4% à 0,5% en poids de probiotique *Lactobacillus rhamnosus,* la souche étant de préférence *Lactobacillus rhamnosus GG,*
pour 100% en poids de la composition ou pour 100% en poids de la somme de la glutamine, curcumine et *Lactobacillus rhamnosus.*

Dans un autre aspect particulier, la composition comprend ou consiste essentiellement en :
- de 90% à 96% en poids de glutamine, de préférence 90% à 95% en poids de glutamine,
- de 3% à 10%, de préférence de 3% à 6%, de manière toute préférée de 4% à 5 % en poids d'extrait de curcuma,
- de 0,25% à 5%, de préférence de 0,3% à 1%, préférentiellement de 0,3% à 0,6%, de manière toute préférée de 0,4% à 0,5% en poids de probiotique *Lactobacillus rhamnosus,* la souche étant de préférence *Lactobacillus rhamnosus GG,*
pour 100% en poids de la composition ou pour 100% en poids de la somme de la glutamine, curcumine et *Lactobacillus rhamnosus.*

Dans un autre aspect particulier, la composition selon l'invention peut comprendre ou consister essentiellement en :
- de 93% à 96% en poids de glutamine, de préférence 93% à 95% en poids de glutamine,
- de 3% à 10%, de préférence de 3% à 6%, de manière toute préférée de 4% à 5 % en poids d'extrait de curcuma,
- de 0,25% à 5%, de préférence de 0,3% à 1%, préférentiellement de 0,3% à 0,6%, de manière toute préférée de 0,4% à 0,5% en poids de probiotique *Lactobacillus rhamnosus,* la souche étant de préférence *Lactobacillus rhamnosus GG,*
pour 100% en poids de la composition ou pour 100% en poids de la somme de la glutamine, curcumine et *Lactobacillus rhamnosus.*

D'autres exemples de proportions seront plus particulièrement décrits ci-après pour chaque composé. En particulier, la composition selon l'invention peut comprendre les différents ingrédients dans les proportions décrites ci-après dans les paragraphes décrits ci-après, et toute combinaison de ces ingrédients et de leurs proportions respectives.

Sauf mention contraire, les proportions exprimées en % correspondent à des pourcentages massiques par rapport au poids total de l'entité considérée (e.g. la composition pharmaceutique, nutraceutique ou alimentaire).

La composition peut en outre comprendre d'autres composés ou ingrédients comme des additifs ou excipients tels que décrits ci-après. De préférence, le pourcentage en poids de ces ingrédients n'excède pas 50%, 40%, 30%, 20%, 10% ou 5% en poids total de la composition, c'est-à-dire en poids de la combinaison des trois ingrédients glutamine, extrait de curcuma et *Lactobacillus rhamnosus,* additionnée avec les autres ingrédients tels que les excipients.

Dans un mode de réalisation, les ratios de ces trois actifs (Glutamine/ *L. rhamnosus,* Glutamine/ Extrait de curcuma et Extrait de curcuma / *L. rhamnosus*) sont compris respectivement entre 200 pour 1 (200 :1), 20 pour 1 (20 :1) et 10 pour 1 (10 :1), respectivement. Ainsi toutes formulations qui contiendraient jusqu'à 10 fois plus d'extrait de curcuma que de *L. rhamnosus,* et/ou jusqu'à 20 fois plus de glutamine que d'extrait de curcuma et/ou jusqu'à 200 fois plus de glutamine que de *L. rhamnosus* rentreraient dans le champ de la présente invention.

Selon un mode de réalisation, la composition comprend les trois ingrédients (glutamine, extrait de curcuma et *Lactobacillus rhamnosus)* dans la même composition. Alternativement, ces trois composés peuvent être compris dans des compositions destinées à être administrées de façon séparée, par exemple dans deux gélules différentes, mais toujours dans les proportions telles que décrites ci-dessus.

### Glutamine

La glutamine est un acide aminé réglementairement considéré comme une substance nutritionnelle et/ou physiologique. De préférence, la composition selon l'invention comprend de la glutamine synthétique, de préférence sans allergènes, préférentiellement de numéro CAS 56-85-9. Dans un mode de réalisation particulier, la composition selon l'invention comprend de la L-glutamine, préférentiellement sous forme de poudre.

La composition divulguée contient de 50 à 95 %, de 50 à 90 %, de 50 à 85 %, de 50 à 80 %, de 50 à 75 %, de 50 à 70 %, de 50 à 65 %, de 50 à 60 %, de 50 à 55 %, de 55 à 95 %, de 55 à 90 %, de 55 à 85 %, de 55 à 80 %, de 55 à 75 %, de 55 à 70 %, de 55 à 65 %, de 55 à 60 %, de 60 à 95 %, de 60 à 90 %, de 60 à 85 %, de 60 à 80 %, de 60 à 75 %, de 60 à 70 %, de 60 à 65 %, de 65 à 95 %, de 65 à 90 %, de 65 à 85 %, de 65 à 80 %, de 65 à 75 %, de 65 à 70 %, de 70 à 95 %, de 70 à 90 %, de 70 à 85 %, de 70 à 80 %, de 70 à 75 %, de 75 à 95 %, de 75 à 90 %, de 75 à 85 %, de 75 à 80 %, de 80 à 95 %, de 80 à 90 %, de 80 à 85 %, 85 à 95 %, 85 à 90 %, de 90 à 95%, de 90% à 96%, ou de 90% à 97% de glutamine telle que définie précédemment, de préférence pour 100% en poids de la somme des trois ingrédients : glutamine, extrait de curcuma et *Lactobacillus rhamnosus* ou pour 100 % en poids de la composition. Préférentiellement, la composition divulguée contient de 70% à 95%, de 75% à 95%, de 85% à 95 %, de 90% à 95%, de 70% à 96%, de 75% à 96%, de 85% à 96 %, de 90% à 96%, de 70% à 97%, de 75% à 97%, de 85% à 97 %, de 90% à 97%, de poudre de glutamine pour 100% en poids des trois ingrédients : glutamine, extrait de curcuma et *Lactobacillus rhamnosus* ou pour 100 % en poids de la composition.

Selon un mode de réalisation très particulier, la composition selon la présente invention contient entre 93% et 96 % ou entre 93% et 95%, tout particulièrement entre 94% et 95%, de glutamine pour 100% en poids des trois ingrédients (glutamine, extrait de curcuma et *Lactobacillus rhamnosus)* ou pour 100% en poids total de la composition.

La dose journalière de glutamine peut être comprise entre 0,5 et 10 g.

### Extrait de Curcuma

Le *Curcuma longa L.,* encore appelé curcumin, curcuma ou curcumine, est une plante vivace originaire d'Asie qui appartient à la famille des Zingiberacées. Le rhizome de curcumin est constitué de mucilages, de polysaccharides, d'huiles essentielles, de polyphénols, et de curcuminoïdes, donnant notamment la couleur jaune /orange au rhizome. On entend par « curcuminoïdes » des composés de type diarylheptanoïdes comprenant notamment la curcumine (N° CAS 458-37-7) et ses dérivés tels que la déméthoxycurcumine (N° CAS 22608-11-3) ou encore la bis-démethoxycurcumine (N° CAS 33171-05-0). La curcumine (1,7-bis (4-hydroxy-3-méthoxyphényl)-1,6-heptadiène-3,5-dione), également appelée diféruloylméthane (N° CAS : 458-37-7), est le curcuminoïde principal du curcuma asiatique.

L'extrait de curcuma présent dans la combinaison est typiquement un extrait de rhizome, sec ou frais, Curcuma longa L. Il peut s'agir d'un extrait standardisé en curcuminoïdes. La composition selon la présente invention peut contenir un extrait de curcuma standardisé en curcuminoïdes. Au sens de l'invention, on entend par le terme « standardisé » ou « standardiser », la notion de contrôle d'un extrait, d'une huile ou tout autre ingrédient afin de le rendre conforme à une norme ou à une teneur minimale définie en certaines molécules, comme par exemple les curcuminoïdes dans le cas de l'extrait de curcuma. Un extrait de curcuma standardisé en curcuminoïdes est donc un extrait de curcuma présentant une teneur minimale définie en curcuminoïdes de 15%.

L'extrait de curcuma présent dans la combinaison selon l'invention peut être obtenu par toute méthode d'extraction connue par exemple par extraction à l'aide d'un solvant organique, par macération ou décoction ou encore par extraction par fluide supercritique. De préférence il s'agit d'un extrait de rhizome de curcuma obtenu par un procédé comprenant une étape d'extraction avec un solvant utilisable dans la préparation de compléments alimentaires, par exemple un alcool ou un ester de type acétate d'éthyle.

De manière avantageuse, l'extrait de curcuma standardisé en curcuminoïdes est utilisé sous forme de poudre. Sa teneur en eau est de préférence inférieure à 15% par rapport au poids total de l'extrait. L'extrait de curcuma contient au minimum 15% ou 20% de curcuminoïdes. De préférence encore, les curcuminoïdes de l'extrait sont répartis ainsi : entre 65 et 82% de curcumine, 15 à 25 % de déméthoxycurcumine et/ou 2 à 7% de bisdéméthoxycurcumine.

Selon un mode réalisation, l'extrait de curcuma comprend de la curcumine numéro CAS 458-37-7, de la déméthoxycurcumine numéro CAS 22608-11-3, et de la bisdéméthoxycurcumine numéro CAS 33171-05-0.

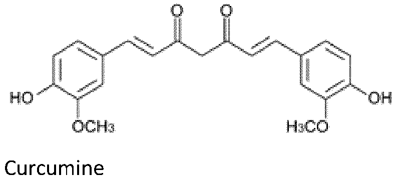

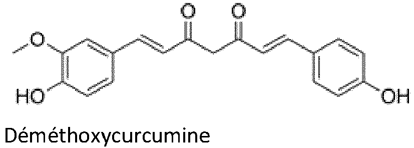

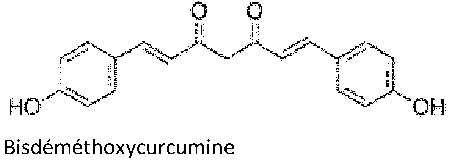

Afin d'améliorer la biodisponibilité des curcuminoïdes, l'extrait de curcuma peut être formulé à l'aide d'un vecteur ou d'un système d'encapsulation tels que des liposomes ou des molécules cages. L'extrait de curcuma est associé à une cyclodextrine, de préférence une gamma-cyclodextrine. Il peut s'agir notamment du produit CAVACURMIN^{®} commercialisé par Wacker.

Dans un mode de réalisation particulier, l'extrait de curcuma est encapsulé avec de la gamma-cyclodextrine, en particulier telle que définie sous le numéro CAS 17465-86-0. L'extrait de curcuma est encapsulé dans une cyclodextrine, de préférence une gamma cyclodextrine, et/ou, dans un aspect non revendiqué, l'extrait de curcuma est associé à un système d'encapsulation ou de vectorisation, de préférence une cyclodextrine.

Dans un mode de réalisation très particulier :
- l'extrait de curcuma comprend au moins 15% en poids de curcuminoïdes, et/ou
- l'extrait de curcuma comprend de la curcumine, de la déméthoxycurcumine et de la bisdéméthoxycurcumine, et/ou
- l'extrait de curcuma comprend au moins 55%, de préférence au moins 60% en poids de curcumine par rapport au poids total des curcuminoïdes présents dans l'extrait, et/ou
- l'extrait de curcuma est encapsulé dans une cyclodextrine, de préférence une gamma cyclodextrine, et/ou
- l'extrait de curcuma est associé à un système d'encapsulation ou de vectorisation, de préférence une cyclodextrine.

Selon un aspect, la composition comprend ainsi de la curcumine, introduite dans la composition par l'ajout d'un extrait de curcuma ou sous une forme purifiée.

La teneur en extrait de curcuma de la composition selon l'invention est avantageusement inférieure à 15% pour 100% en poids des trois ingrédients (glutamine, extrait de curcuma et *Lactobacillus rhamnosus)* ou pour 100 % en poids de la composition. La teneur en extrait de curcuma de la composition divulguée est de préférence de 1 % à 15 %, de 2 % à 15 %, de 3 % à 15 %, de 4 % à 15 %, de 5 % à 15 %, de 6 % à 15 %, de 7 % à 15 %, de 8 % à 15 %, de 9 % à 15 % ou de 10 % à 15 %, préférentiellement 1 % à 10%, de 2 % à 10 %, de 3 % à 10 %, de 4 % à 10 % ou de 5 % à 10 %, et plus préférentiellement encore de 1 % à 5 %, de 2 % à 5 %, de 3 % à 5 % ou de 4 % à 5 %, de manière toute préférée de 4%, 4,1%, 4,3% , 4,4%, 4,5%,4,6% 4,7% ,4,8% ou 4,9 % pour 100% en poids des trois ingrédients (glutamine, extrait de curcuma et *Lactobacillus rhamnosus)* ou pour 100 % en poids de la composition.

Préférentiellement, la composition selon la présente divulgation contient de 3% à 10%, de préférence 4 % à 9 %, de curcuma pour 100% en poids des trois ingrédients : glutamine, extrait de curcuma et *Lactobacillus rhamnosus* ou pour 100 % en poids de la composition.

Selon un aspect non revendiqué, la composition selon la présente divulgation contient de 4% à 8%, de curcuma pour 100% en poids total de la composition.

Selon un autre mode de réalisation très particulier, la composition selon la présente invention contient de 4% à 5%, de curcuma pour 100% en poids des trois ingrédients (glutamine, extrait de curcuma et *Lactobacillus rhamnosus)* ou pour 100 % en poids de la composition.

La dose journalière d'extrait de curcuma peut être comprise entre 25 mg et 500 mg.

### Bactérie Lactobacillus rhamnosus

Certaines des souches probiotiques les plus étudiées appartiennent au groupe des bactéries lactiques (LAB). Ces bactéries sont des microorganismes Gram-positif, à faible contenu en génome GC, anaérobies ou aérotolérantes, généralement non sporulées, qui produisent de l'acide lactique comme principal produit final de la fermentation des glucides. Les LAB sont généralement reconnues comme sûres et certaines espèces ont obtenu le statut de présomption de sécurité (QPS) de l'Agence européenne de sécurité des aliments (EFSA).

La souche bactérienne utilisée dans la composition selon l'invention est une souche de bactérie de l'espèce *Lactobacillus rhamnosus,* en particulier sélectionnée dans le groupe constitué par *L. rhamnosus* GG (ATCC 53103), *L. rhamnosus* Lc705 (DSM 7061), *L. rhamnosus* CNCM I-3690, *L. rhamnosus* CNCM I-1720, *L. rhamnosus* la801, *L. rhamnosus* sp1, *L. rhamnosus* HN001 (NM97/09514), *L. rhamnosus* r0011, *L. rhamnosus* r0052, *L. rhamnosus* ha-111, *L. rhamnosus* jb-1, *L. rhamnosus* la801, *L. rhamnosus* Ir06, *L. rhamnosus* Ir-32, *L. rhamnosus* Ibv96 et *L. rhamnosus* Icr35.

De préférence, la souche bactérienne est *Lactobacillus rhamnosus* GG. Cette souche a été déposée sous le Traité de Budapest auprès l'American Type Culture Collection (ATCC) sous le numéro d'accession ATCC 53103.

Il est entendu que la présente invention concerne également l'utilisation de toute bactérie issue de l'espèce *Lactobacillus rhamnosus,* ainsi que l'utilisation de toute souche ou d'homologues issus de cette espèce bactérienne. Les termes « homologue », « variant » ou « mutant » sont interchangeables et réfèrent à une souche bactérienne ayant une homologie ou une identité de séquence avec la séquence nucléotidique de la souche bactérienne parente (la séquence de référence). Ceci inclut des souches bactériennes ayant au moins 95%, 96%, 97%, 98% ou 99% d'identité avec la séquence nucléotidique du génome de l'espèce *Lactobacillus rhamnosus,* de préférence avec la séquence nucléotidique du génome de la souche *Lactobacillus rhamnosus GG.* Les mutants peuvent être obtenus par des techniques de génie génétique permettant d'inférer l'altération du matériel génétique des souches de l'invention ou d'inférer une recombinaison du matériel génétique des souches de l'invention avec d'autres molécules. Typiquement, afin d'obtenir de telles souches mutantes, l'homme du métier peut utiliser des techniques de mutagenèse standard telles qu'un rayonnement UV ou une exposition à des produits chimiques mutagènes.

La teneur en *Lactobacillus rhamnosus* de la composition divulguée est avantageusement de 0,02% à 5 %, de 0,05% à 5 %, de 0,1% à 5 %, de 0,25% à 5 %, de 0,5% à 5%, de 0,5% à 4%, de 0,5% à 3%, de 0,5% à 2,5%, de 0,5% à 2%, de 0,5% à 1,5% ou de 0,5% à 1%, préférentiellement de 0,5 à 1,5 %, de 0,5 à 1,25 % ou de 0,5 à 0,75%, plus préférentiellement encore inférieure à 1%, et de manière toute préférée de 0,5%, 0,6%, 0,7 % ou de 0,8%, pour 100% en poids des trois ingrédients (glutamine, extrait de curcuma et *Lactobacillus rhamnosus*) ou pour 100% en poids de la composition.

La composition selon la présente invention contient de 0,25% à 5%, de préférence 0,3% à 4%, de *Lactobacillus rhamnosus* pour 100% en poids des trois ingrédients : glutamine, extrait de curcuma et *Lactobacillus rhamnosus* ou pour 100 % en poids de la composition.

Selon un mode de réalisation très particulier, la composition selon la présente invention contient 0,3% à 0,6%, de préférence 0,4% à 0,5%, de *Lactobacillus rhamnosus* pour 100% en poids des trois ingrédients (glutamine, extrait de curcuma et *Lactobacillus rhamnosus)* ou pour 100 % en poids de la composition.

Les bactéries contenues dans la composition selon l'invention peuvent être vivantes ou inactivées. De préférence, les bactéries contenues dans la composition sont sous forme vivante. Elles peuvent notamment être sous une forme hydratée, lyophilisée, congelée, atomisée ou toute autre forme leur permettant d'être administrées vivantes au sujet. Celles-ci peuvent être administrées telles quelles ou après reprise dans un véhicule physiologiquement acceptable approprié.

Les bactéries sont administrées, par exemple, sous la forme de bactéries vivantes cultivées, sous forme végétative. Alternativement, les bactéries sont fournies sous forme de populations purifiées obtenues à partir d'un matériau microbiotique tel qu'un matériau fécal. Par vivante, il faut comprendre que l'intégrité de la cellule est conservée et que les processus cellulaires se produisent ou peuvent se produire si la bactérie est cultivée dans un milieu et des conditions adéquates. Toute bactérie vivante peut être de nouveau ensemencée sur un milieu de culture adéquat et se multiplier, dans des conditions adéquates.

La composition selon l'invention contient de préférence entre 10⁷ à 10¹¹, notamment de 10⁸ à 10¹¹, de préférence de 10⁹ à 10¹¹ cellules formant des colonies (CFU), de préférence de cellules de *Lactobacillus rhamnosus* par gramme de composition. Le terme « CFU » signifie « colony forming units » selon l'expression technique anglo-saxonne. Par gramme de composition, on entend de préférence la composition selon l'invention comprenant les bactéries selon l'invention, et les co-ingrédients, excipients ou véhicules appropriés.

La dose journalière de *Lactobacillus rhamnosus* peut être comprise entre 0,05 mg et 500 mg ou entre 1x10⁷ CFU et 1x10¹¹ CFU.

### Ingrédients supplémentaires

La composition peut comprendre en outre :
- un ou plusieurs prébiotiques ; et/ou
- des vitamines, minéraux et/ou oligo-élements ; et/ou
- des poudres ou extraits de plantes, fruits, légumes, algues ou champignons ; et/ou
- un ou plusieurs probiotiques.

La composition pharmaceutique, nutraceutique ou alimentaire peut également comprendre une ou plusieurs souches additionnelles de microorganismes, notamment des microorganismes également utilisés comme probiotiques. A titre d'exemples, la composition peut comprendre une ou plusieurs souches additionnelles de microorganisme choisies dans le groupe constitué de Bacillus, Bacteroides, Bifidobacterium, Enterobacteriaceae, Enterococcus, Faecalibacterium, Fusobacterium, Kluyveromyces, Lactobacillus, Odoribacter, Parabacteroides, Pediococcus, Ruminococcus, Streptococcus et/ou Saccharomyces.

Dans un autre mode de réalisation, la composition comprend exclusivement des bactéries du genre Lactobacillus. De préférence, la composition ne comprend pas de bactéries du genre Bifidobacterium et/ou d'une espèce de Lactobacillus autre que rhamnosus. En particulier, la composition comprend comme probiotique des bactéries de l'espèce *Lactobacillus rhamnosus à* l'exclusion de toute autre microorganisme, en particulier toute autre bactérie probiotique et/ou levure.

Dans un mode de réalisation particulier, la composition pharmaceutique, nutraceutique ou alimentaire comprend moins de 20 souches de microorganismes différentes, de préférence moins de 10, 9, 8, 7, 6, 5, 4 ou 3 souches de microorganismes différentes.

Par ailleurs ou en outre, la composition pharmaceutique, nutraceutique ou alimentaire peut également comprendre un ou plusieurs prébiotiques.

Selon un mode de réalisation, la composition pharmaceutique, nutraceutique ou alimentaire selon l'invention comprend un probiotique sous forme vivante ainsi qu'un ou plusieurs prébiotiques pouvant être dégradés par le probiotique ou le microbiote intestinal. Cette association de prébiotique(s) et probiotique constitue un symbiotique.

Selon un aspect particulier, la composition selon l'invention peut comprendre en outre :
- des vitamines, des minéraux ou des oligo-élements ;
- des poudres ou extraits (secs ou liquides) de plantes, fruits, légumes, algues ou champignons ; et/ou
- des substances physiologiques à but nutritionnel ou santé, notamment des prébiotiques, de préférence telles que définies dans l'Arrêté du 26 septembre 2016 établissant la liste des substances à but nutritionnel ou physiologique autorisées dans les compléments alimentaires et les conditions de leur emploi.

Les vitamines, minéraux et/ou oligo-éléments peuvent par exemple être choisis parmi: une vitamine (la thiamine (B1), la riboflavine (B2), la niacine (B3), l'acide pantothénique (B5), la pyridoxine (B6), l'acide folique (B9) et la cyanocobalamine (B12), ainsi que les vitamines C, A, D, E, K1 et K2, un minéral tel que le magnésium, le calcium, ou le fer, des oligoéléments comme l'iode, le fer, le cuivre, le zinc, le sélénium, le chrome, le molybdène, le bore, le manganèse, ou un de leurs mélanges.

Les poudres ou extraits (secs ou liquides) de plantes, fruits, légumes, algues ou champignons peuvent notamment être choisies parmi les différentes listes réglementaires européennes connues de l'homme du métier comprenant par exemple : poudre/extrait de konjac, poudre/extrait d'ignames, poudre/extrait de haricots, poudre/extrait de café, poudre/extrait de thé, poudre/extrait d'écorce de pin, poudre/extrait de raisin, poudre/extrait d'ail, poudre/extrait de safran poudre/extrait de pomme, poudre/extrait de reishi, poudre/extrait de spiruline, poudre/extrait de chlorelles, poudre/ extrait d'algues brunes et tout mélange de ceux-ci.

Dans certains modes de réalisation, la substance physiologique à but nutritionnel ou santé est un prébiotique. Le prébiotique peut particulièrement être un polymère de glucides plus ou moins ramifié et de degré de polymérisation variable. Le prébiotique peut par exemple être choisi parmi l'inuline, l'inositol, le tagatose, le lactulose, l'alphaglucane oligosaccharide, les transgalacto-oligosaccharides (TOS), les fructo-oligosaccarides (FOS), les galacto-oligosaccharides (GOS), les xylo-oligosaccharides (XOS) et un de leurs mélanges.

Dans certains modes de réalisation, la substance physiologique à but nutritionnel ou santé est un acide aminé comme, sans s'y limiter, la leucine, la citrulline, la glutamine, le glutamate, la cystéine et ses dérivés, la méthionine et ses dérivés, le tryptophane et ses dérivés.

Dans certains modes de réalisation, la substance physiologique à but nutritionnel ou santé est un antioxydant comme par exemple, sans s'y limiter : la super oxyde dismutase (SOD), l'ubiquinol/ubiquinone (Coenzyme Q10), le resvératrol, les catéchines (catéchine, épicatéchine et dérivés gallatés), les flavanols, les flavanones ou encore les anthocyanes.

### Composition nutraceutique

Selon un aspect particulier, la composition est une composition nutraceutique. On entend par « composition nutraceutique » toute composition comprenant des ingrédients alimentaires tels que des macronutriments, des micronutriments, des plantes ou extraits de plantes, ou les substances ayant un effet nutritionnel ou physiologique, visant à compléter le régime alimentaire d'un être humain afin d'améliorer le statut nutritionnel de ce dernier et favoriser ainsi un bon état de santé. Tels qu'envisagées ici, les produits nutraceutiques sont des nutriments isolés, des compléments alimentaires ou sont inclus dans certains aliments destinés à des groupes spécifiques. Les compositions nutraceutiques englobent ainsi des compositions comprenant de la glutamine, de l'extrait de curcuma et des bactéries probiotiques *Lactobacillus rhamnosus* et un composant de qualité alimentaire, en particulier un additif ou auxiliaire technologique.

### Composition alimentaire

Selon un aspect particulier, la composition est une composition alimentaire. On entend par « composition alimentaire » toute composition comprenant des ingrédients alimentaires tels que des macronutriments, des micronutriments, des vitamines, des minéraux, oligo-éléments ou des substances ayant un effet nutritionnel ou physiologique. Tels qu'envisagées ici, les produits alimentaires sont des aliments conventionnels, des aliments fonctionnels ou des aliments transformés. Ainsi, par définition, les compositions alimentaires peuvent être des aliments divers et variés, connus de l'homme du métier.

La composition alimentaire peut être notamment un aliment destiné à l'alimentation humaine ou animale qui pourra être un liquide, une pâte ou un solide. A titre d'exemples, sans s'y limiter, l'aliment pourra être un produit laitier tel que du fromage, du beurre, une crème, les yaourts, les glaces, les fromages, les produits cuits tels que le pain, les biscuits et les gâteaux, un produit à base de fruit comme un jus de fruit, une compote ou une pâte de fruit, les produits alimentaires à base de soja, les produits alimentaires à base d'amidon, produits de confiserie, compositions pour huiles comestibles, pâtes à tartiner, céréales pour petit déjeuner, préparations pour nourrissons, jus, les barres alimentaires (par exemple les barres de céréales, les barres de petit-déjeuner, les barres énergétiques, les barres nutritionnelles); biscuits, snack, chewing-gums; des boissons; boissons énergisantes, boissons enrichies; suppléments à boire (des poudres à ajouter à une boisson); etc.

Selon un aspect particulier, la composition alimentaire n'est pas à base de lait. De préférence, la composition alimentaire ne comprend pas de protéine(s) ou d'oligosaccharide(s) du lait humain ou animal.

Ainsi, la composition alimentaire selon l'invention comprend de la glutamine, un extrait de curcuma et la bactérie *Lactobacillus rhamnosus* et les composants de l'un au moins des aliments ou des boissons précédemment cités.

L'invention se rapporte également à une méthode de préparation d'une composition nutraceutique comprenant l'ajout de glutamine, d'extrait de curcuma et d'une souche de *Lactobacillus rhamnosus,* en particulier en tant que principe actif, aux composants de la composition nutraceutique. L'invention se rapporte en outre à une méthode de préparation d'une composition alimentaire comprenant l'ajout de glutamine, d'extrait de curcuma et d'une souche de *Lactobacillus rhamnosus,* en particulier en tant que principe actif, aux composants de la composition alimentaire.

L'invention concerne aussi une méthode pour améliorer le bien-être mental ou diminuer les troubles de l'humeur d'un sujet comprenant l'administration d'une composition nutraceutique ou alimentaire comprenant une quantité efficace de glutamine, d'extrait de curcuma et d'une souche de *Lactobacillus rhamnosus.*

L'invention se rapporte, par ailleurs, à une utilisation non thérapeutique de la composition nutraceutique ou alimentaire selon l'invention telle que définie précédemment pour la préparation de compositions destinées à améliorer le bien-être mental d'un sujet.

### Composition pharmaceutique et médicament

La composition selon l'invention peut en particulier être une composition pharmaceutique destinée à un usage en médecine humaine ou un usage vétérinaire.

La composition pharmaceutique selon l'invention comprend une combinaison de glutamine, d'extrait de curcuma et d'une souche de *Lactobacillus rhamnosus* en tant que principe actif. Dans un mode de réalisation, la composition pharmaceutique peut, en outre, contenir au moins un principe actif pharmaceutique supplémentaire. On entend par « principe actif pharmaceutique », tout composé ou substance dont l'administration a un effet thérapeutique ou un effet bénéfique à la santé ou condition générale d'un patient ou d'un sujet auquel il est administré. Dans un mode de réalisation, l'autre principe actif pharmaceutique n'est pas une bactérie probiotique, en particulier une souche de Bifidobactéries et/ou une espèce de Lactobacillus autre que rhamnosus. Par exemple, le ou les principes actifs supplémentaires pourraient être un médicament antidépresseur ou un médicament anxiolytique. Alternativement, l'autre principe actif pharmaceutique contenu dans la composition pharmaceutique est une souche probiotique ou une combinaison de souches probiotiques. A titre d'exemples, la composition peut comprendre une ou plusieurs souches additionnelles de microorganisme choisies dans le groupe constitué des bactéries du genre Bacillus, Bacteroides, Bifidobacterium, Enterobacteriaceae, Enterococcus, Faecalibacterium, Fusobacterium, Kluyveromyces, Lactobacillus, Odoribacter, Parabacteroides, Pediococcus, Roseburia, Ruminococcus, Saccharomyces et Streptococcus.

La présente divulgation concerne également une combinaison de glutamine, d'extrait de curcuma et d'une souche de *Lactobacillus rhamnosus* pour une utilisation en tant que médicament, préférentiellement pour une utilisation en tant que médicament dans le domaine de la psychiatrie, en particulier pour la régulation des troubles anxieux et/ou des troubles de l'humeur tels que la dépression, de préférence pour diminuer l'anxiété et/ou la dépression et/ou les symptômes anxio-dépressifs y étant associés.

Particulièrement, la présente invention concerne une composition selon l'invention, de préférence une composition pharmaceutique, pour une utilisation dans le traitement d'un trouble anxieux et/ou de la dépression. De préférence, ledit sujet ayant été préalablement diagnostiqué comme présentant des troubles anxieux et/ou des troubles de l'humeur, en particulier la dépression, ou étant à risque de développer l'un de ces troubles.

La composition selon l'invention peut être utilisée en combinaison avec un autre principe actif, par exemple un médicament antidépresseur ou un médicament anxiolytique. Ainsi, la présente invention est également relative à une composition pour son utilisation dans le traitement d'un trouble anxieux et/ou un trouble de l'humeur, particulièrement la dépression, et/ou les symptômes mentaux et/ou comportementaux y étant associés, en combinaison avec un médicament antidépresseur ou un médicament anxiolytique.

### Formulation de la composition

La formulation d'une composition pharmaceutique, nutraceutique ou alimentaire selon la présente invention peut varier en fonction de la voie d'administration et du dosage pour lesquels la composition est destinée à être utilisée. La composition pharmaceutique, nutraceutique ou alimentaire selon l'invention peut notamment être sous forme solide, semi-solide ou liquide. Après formulation avec au moins un véhicule ou excipient physiologiquement acceptable, une composition selon l'invention peut être sous toute forme appropriée pour l'administration à un mammifère, en particulier l'homme, par exemple sous la forme de tablettes, comprimés, pastilles, dragées, capsules, gélules, pilules, granulats, poudre, suspensions, émulsions, sirops, onguents, ampoule de liquide, flacon muni d'un compte-goutte et autres formes analogues de préparations liquides ou en poudres, ou suppositoires.

L'homme de l'art sait sélectionner les véhicules et excipients les plus appropriés à la préparation d'un type donné de formulation.

Dans un mode de réalisation particulier, le véhicule physiologiquement acceptable peut être un solide et la composition selon l'invention peut être sous la forme d'une poudre ou d'un comprimé. Le véhicule physiologiquement acceptable peut alternativement être un liquide, et la composition selon l'invention est sous la forme d'une solution. Les véhicules liquides sont utilisés dans la préparation de solutions, de solvants, de milieux de dispersion, de suspensions, d'émulsions, de sirops, d'élixirs et de compositions sous pression. Les supports liquides ou à base de gel appropriés comprennent, sans s'y limiter : l'eau et les solutions salines physiologiques ; les émulsions ou les suspensions, y compris les solutions salines et les milieux tamponnés, l'urée ; des alcools (par exemple, éthanol, des solvants non aqueux comme le des huiles végétales ou de graines telles que l'huile d'olive). Les compositions liquides, y compris par exemple les émulsions, microémulsions, solutions, suspensions, sirops, ou élixirs peuvent être formulées notamment en présence de solvants, d'agents solubilisants, d'émulsifiants, d'huiles, d'acides gras, et/ou d'autres additifs comme par exemple des agents de suspension, des conservateurs, des édulcorants, des arômes naturels ou synthétiques, des agents viscosifiants, des agents stabilisants et/ou épaississants et/ou des agents colorants de qualité alimentaire, qui doivent tous être compatibles avec le maintien de la viabilité de la souche *Lactobacillus rhamnosus.* Les émulsions peuvent contenir des agents émulsifiants tels que la lécithine, le monooléate de sorbitan ou l'acacia. Des agents épaississants bien connus peuvent également être ajoutés aux compositions telles que l'amidon de maïs, des gommes naturelles ou synthétiques, des résines, la méthylcellulose, la carboxyméthylcellulose sodique, la gomme de guar, la gomme de xanthane et analogues. La composition de l'excipient peut être modifiée aussi longtemps qu'elle n'interfère pas de manière significative avec l'activité pharmacologique des bactéries probiotiques *Lactobacillus rhamnosus* selon l'invention.

En particulier, la composition selon l'invention peut comprendre en outre :
- des anti-agglomérants comme le carbonate de magnésium, le dioxyde de silice ou l'acide stéarique ;
- un ou plusieurs agents de viscosité ou tensio-actifs, notamment l'amidon, les éthers de cellulose comme l'hydroxypropylméthylcellulose ou la gomme arabique ;
- un ou plusieurs agents liants, notamment l'huile de poisson ou toute huile végétale telle que par exemple l'huile de tournesol, l'huile de soja, l'huile d'olive ou l'huile de chia ;
- des agents d'enrobage comme la cire d'abeille, la cire de canauba ou les monoglycérides d'acides gras alimentaires ;
- des émulsifiants comme la lécithine, les cyclodextrines ou la gomme ester ;
- un ou plusieurs agents édulcorants non nutritifs, notamment le sorbitol, le sucralose, l'aspartame, le néotame, le maltitol, le xylitol, l'acésulfame-K, l'aspartame, la saccharin, l'acide glycyrrhizique ou un extrait de stévia ;
- un ou plusieurs agents de saveur, naturels ou artificiels, sous forme pulvérulente ou liquide ;
- un ou plusieurs correcteurs d'acidité, notamment le sel, l'acide citrique, l'acide malique ou l'acide tartrique ; et/ou
- un ou plusieurs colorants, notamment les anthocyanes, le béta carotène, l'extrait de betterave, le lycopène ou l'extrait de caramel.

Dans certains modes de réalisation, une composition pharmaceutique, nutraceutique ou alimentaire selon la présente invention est formulée pour une libération immédiate du ou des principes actifs. Alternativement, une composition pharmaceutique peut être formulée pour une libération prolongée ou ciblée du ou des principes actifs ou pour une protection du ou des principes actifs, par exemple vis-à-vis de l'acidité et des enzymes gastriques. Il est ainsi possible d'utiliser des enrobages résistants au pH et/ou à l'action des enzymes gastriques, des enrobages sensibles au pH et/ou à des actions enzymatiques, ou des enrobages bioadhésifs qui s'accrochent aux parois de l'estomac ou de l'intestin, ou des systèmes d'encapsulation.

De préférence, la composition selon l'invention est une composition sous une forme adaptée pour une administration par voie orale. Elle peut se présenter notamment sous la forme de pilules, comprimés, gélules, ou poudres éventuellement à dissoudre ou à re-suspendre dans un véhicule adapté, de pâte ou gomme à mâcher, de bonbons à sucer ou à croquer, de solutions, par exemple solutions buvables conditionnées dans des ampoules, de gel, etc.

De préférence, la composition est sous une forme orale gastro-résistante permettant aux bactéries contenues dans la composition selon l'invention de passer l'estomac et d'être libérées dans l'intestin. Un enrobage entérique peut être stable à pH acide (comme dans l'estomac) et peut se dissoudre à un pH basique (par exemple, dans l'intestin). Le matériau qui peut être utilisé dans les enrobages entériques comprend, par exemple, l'acide alginique, l'acétate phtalate de cellulose, les cires, la gomme laque, les acides gras (par exemple l'acide stéarique ou l'acide palmitique) ou encore le chitosan de façon non exclusive.

Dans un mode de réalisation, le revêtement gastro-résistant et entéro-soluble est conçu pour maintenir la stabilité des ingrédients actifs dans l'estomac. Le revêtement entérique est conçu pour se maintenir dans des conditions acides de l'estomac et se dégrader dans des conditions non acides, libérant ainsi la composition pharmaceutique, nutraceutique ou alimentaire dans les intestins.

Les enrobages entériques peuvent être utilisés pour i) empêcher le suc gastrique de réagir avec ou détruire la substance active, ii) empêcher la dilution de la substance active avant qu'elle n'atteigne l'intestin, iii) s'assurer que la substance active n'est libérée qu'après le passage de la préparation à travers l'estomac, et iv) empêcher les bactéries *Lactobacillus rhamnosus* vivantes contenues dans la composition selon l'invention d'être altérées ou tuées par le pH acide de l'estomac.

Selon un mode de réalisation, la composition peut comprendre en outre de la maltodextrine et/ou du stéarate de magnésium. En particulier, la composition peut être formulée selon l'un des exemples décrits ci-après.

Dans un mode de réalisation particulier, la composition selon l'invention se présente sous la forme d'unités de dose comprenant :
- entre 0,5 g et 10 g, de préférence entre 0,5 g et 2 g de glutamine,
- entre 25 mg et 500 mg, de préférence entre 50 mg et 100 mg d'extrait de curcuma, et
- entre 0,05 mg et 500 mg, de préférence entre 10 mg et 20 mg, ou entre 1x10⁷ CFU et 1x10¹¹ CFU de *Lactobacillus rhamnosus,* de préférence *Lactobacillus rhamnosus GG.*

Les unités de dose peuvent être par exemple des comprimés et des gélules.

Dans un mode de réalisation particulier, les unités de doses sont comprises dans un sachet unique présentant deux compartiments. Le premier compartiment comprend les bactéries *Lactobacillus rhamnosus,* et l'autre compartiment comprend la glutamine et l'extrait de curcuma, par exemple sous forme de poudres.

Un objet supplémentaire selon l'invention est un kit d'administration, un kit nutraceutique ou pharmaceutique, par exemple un kit de complément alimentaire comprenant plusieurs compositions, de préférence destinées à l'administration orale, intégrant la combinaison selon l'invention.

L'invention a ainsi également pour objet un kit de préférence, pharmaceutique, nutraceutique ou alimentaire, comprenant l'association de glutamine, d'un extrait de curcuma et de Lactobacillus rhamnosus, ledit kit étant adapté(e) aux sujets souffrant ou susceptibles de troubles des émotions.

Dans un mode de réalisation particulier, ledit kit comprend :
- une composition comprenant la bactérie *Lactobacillus rhamnosus,*
- une composition comprenant la glutamine, et
- une composition comprenant l'extrait de curcuma.

Par exemple, la glutamine, l'extrait de curcuma et la bactérie de *Lactobacillus rhamnosus* peuvent être administrés sous la forme d'un kit c'est-à-dire sous la forme de plusieurs compositions pharmaceutiques, nutraceutiques ou alimentaires distinctes, par exemple sous la forme d'aux moins deux unités de dose pour l'administration orale, par exemple d'une première gélule contenant Lactobacillus rhamnosus et d'un comprimé ou d'une deuxième gélule contenant la glutamine et l'extrait de curcuma.

Dans un mode de réalisation particulier, le kit d'administration peut prendre la forme d'un sachet unique présentant deux compartiments destinés à recevoir les différents ingrédients de la composition selon l'invention. De préférence, le premier compartiment comprend les bactéries *Lactobacillus rhamnosus,* et l'autre compartiment comprend la glutamine et l'extrait de curcuma.

Le kit d'administration de la combinaison peut alternativement comprendre trois gélules distinctes, une gélule contenant les bactéries, un comprimé ou gélule contenant la glutamine, et un comprimé ou gélule contenant le l'extrait de curcuma.

Dans un mode de réalisation particulier, le kit d'administration peut prendre la forme d'un sachet unique présentant trois compartiments destinés à recevoir chacun des trois différents ingrédients de la composition selon l'invention séparément.

Dans un mode particulier de réalisation, le kit comprend :
- une première composition comprenant la bactérie *Lactobacillus rhamnosus,* par exemple associée à un ou plusieurs excipients,
- une seconde composition comprenant la glutamine et l'extrait de curcuma, par exemple associée à un ou plusieurs excipients,

Chacune de ces deux compositions peut être sous la forme de gélule, de comprimé ou de poudre.

Le kit comprend typiquement plusieurs unités de dose de chaque composition. Il peut comprendre également des instructions d'administration du kit.

Dans un mode de réalisation particulier, la première composition peut se présenter sous la forme d'unités de dose comprenant entre 0,5 et 10 g de glutamine, entre 25 mg et 500 mg d'extrait de curcuma et entre 0,05 mg et 500 mg ou entre 1x10⁷ CFU et 1x10¹¹ CFU de *Lactobacillus rhamnosus.* Les unités de dose peuvent être par exemple des comprimés et des gélules.

Les caractéristiques des compositions présentes dans ce kit sont similaires à celles de la composition selon l'invention notamment, en termes de rapport massique de Lactobacillus rhamnosus, glutamine et extrait de curcuma entre eux.

Chaque composition présente dans le kit peut comprendre un ou plusieurs ingrédients supplémentaires choisis parmi les additifs, excipients et actifs supplémentaires acceptables sur le plan pharmaceutique, nutraceutique et/ou alimentaire tels que décrits ci-avant.

### Sujet et régime d'administration

La composition pharmaceutique, nutraceutique ou alimentaire selon l'invention peut être employée à des fins médicales ou d'alimentation humaine ou animale, le sujet considéré pouvant notamment être un mammifère, plus particulièrement tout sujet animal comme un animal de laboratoire (par exemple, primate non humain, rat, souris, hamster, cobaye), du bétail (par exemple, vache, mouton, chèvre, porc, dinde et poulet) ou une espèce domestique (par exemple chien, chat et rongeur), et plus particulièrement encore un humain, par exemple un enfant ou un adulte.

Par enfant, on entend selon l'invention un individu dont l'âge est inférieur à 18 ans. Sont ainsi compris dans la catégorie des enfants selon l'invention, les nouveau-nés, dont l'âge est compris entre 0 et 1 mois, les nourrissons, qui ont entre 1 mois et 2 ans, et les enfants proprement dits, qui sont âgés d'au moins 2 ans. Par adulte, on entend toute personne âgée d'au moins 18 ans.

Les compositions pharmaceutiques, nutraceutiques ou alimentaires selon la présente invention peuvent être administrées en utilisant toute combinaison de dosage et de voie d'administration efficace pour obtenir l'effet thérapeutique désiré. La quantité exacte à administrer et la fréquence d'administration dépendront notamment du type de sujet, humain ou animal, en fonction de l'âge, du poids, de la condition générale du patient ou de l'animal, de la nature et de la gravité du trouble. La voie d'administration peut être choisie en fonction de l'intensité du trouble et/ou en fonction de l'âge et/ou de la santé du patient.

Dans un aspect particulier, pour un adulte, la composition est destinée à être administrée de telle sorte que la dose journalière de la composition soit comprise entre 0,5 et 10 g, de préférence entre 1 et 5 g. Notamment, la dose journalière de glutamine peut être comprise entre 0,5 et 10 g, et/ou la dose journalière d'extrait de curcuma peut être comprise entre 25 mg et 500 mg, et/ou la dose journalière de *Lactobacillus rhamnosus* peut être comprise entre 0,05 mg et 50 mg ou entre 1x10⁷ CFU et 1x10¹¹ CFU.

La composition selon l'invention peut être administrée en une ou plusieurs fois, c'est-à-dire sous la forme d'une dose unique ou de doses multiples.

Une dose peut représenter plusieurs milligrammes à plusieurs dizaines de grammes de composition selon l'invention. Typiquement, une dose représente entre 1 mg et 100 mg, entre 1 mg et 1 g, entre 1 mg et 10 g, entre 10 mg et 100 mg, entre 10 mg et 1 g, entre 10 mg et 10 g, entre 100 mg et 1g, entre 100 mg et 10 g ou entre 1 g et 10 g de composition selon l'invention, de préférence entre 100 mg et 10 g de composition selon l'invention, et de manière plus particulièrement préférée entre 500 mg et 5 g de composition selon l'invention.

La composition selon l'invention peut être administrée à un sujet avant l'apparition de symptômes (c'est-à-dire de manière prophylactique, par exemple, avant l'apparition d'une situation génératrice d'anxiété) ou après l'apparition de symptômes aigus ou chroniques (c'est-à-dire à visée thérapeutique, par exemple, après apparition de symptômes anxio-dépressifs). La composition selon l'invention peut ainsi être administrée à tout moment et à n'importe quel intervalle. Ainsi, dans certains modes de réalisation, l'administration de la composition pharmaceutique, nutraceutique ou alimentaire, du médicament, du complément alimentaire ou de l'aliment selon l'invention est épisodique.

Dans un autre mode de réalisation, l'administration de la composition, du médicament, du complément alimentaire ou de l'aliment selon l'invention est à intervalles réguliers. De préférence, la composition est administrée à un sujet à une fréquence comprise entre une dose par jour et une dose par mois. Typiquement, la fréquence d'administration est comprise entre une ou plusieurs doses par jour et une dose par semaine, par exemple une dose tous les 2, 3, 4, 5, 6 ou 7 jours. Alternativement, la fréquence d'administration peut être de plusieurs doses par jour, par exemple 2, 3, 4 ou 5 doses par jour, ou encore en une seule dose (mono-dose). Selon l'âge du sujet ou son état physiologique, les doses journalières peuvent être fractionnées pour faciliter l'administration, par exemple avec une administration le matin et une autre le soir.

Dans un mode de réalisation, la composition est administrée pendant environ 24 heures, environ 2 jours, environ 5 jours, environ 10 jours, environ 15 jours, environ 30 jours ou 1 mois, environ 2 mois, environ 4 mois, environ 6 mois, ou environ 1 an après l'apparition initiale des symptômes (par exemple, symptômes associés à l'état dépressif) et/ou après le diagnostic d'un trouble (par exemple une dépression) chez le sujet.

La composition selon l'invention, le médicament, le complément alimentaire ou l'aliment peut être administré par diverses voies, de préférence entérale comprenant, mais sans nécessairement se limiter à l'administration orale ou intranasale. Selon une mise en œuvre particulière de l'invention, la composition, le médicament, le complément alimentaire ou l'aliment est administré par voie orale. Dans un mode de réalisation particulier, le médicament ou la composition nutraceutique ou alimentaire est administré par sonde gastrique.

### Les troubles psychiatriques ciblés par l'invention

La présente divulgation concerne la régulation de l'état émotionnel d'un sujet, la prévention et/ou le traitement des troubles anxieux et/ou d'un trouble de l'humeur tel que la dépression, et/ou des troubles anxio-dépressifs, et/ou les symptômes mentaux et/ou comportementaux y étant associés. Le sujet peut notamment être prédisposé à un tel état ou peut présenter un ou plusieurs symptômes d'un tel état. L'invention concerne également la prévention de l'apparition d'un épisode dépressif ou d'anxiété chez un sujet.

Les troubles de l'humeur, souvent associés à une anxiété, peuvent être tout état psychologique associé à un ou plusieurs symptômes somatiques ou psychosomatiques. Dans certains cas, le développement d'un trouble de l'humeur, en particulier la dépression, est associé ou caractérisé par un symptôme tel que tristesse, irritabilité, agitation, fatigue, perte de poids, des problèmes de concentration, de mémorisation et/ou de prise de décision, un apitoiement, des changements d'appétit ou de poids, une perturbation du sommeil, des sentiments de culpabilité et/ou de désespoir, une dépendance, une diminution de l'énergie, une diminution de l'intérêt ou du plaisir, une indécision, une rumination, un repli sur soi, un manque d'estime de soi, des pensées ou tendances suicidaires, une tension, des sautes d'humeur, des pensées et/ou discours ralentis, des sentiments d'inutilité, une impuissance, de la colère, de l'hostilité, des difficultés à penser, à se concentrer ou à prendre des décisions, et/ou des larmoiements. Ces symptômes peuvent en outre entrainer une manifestation somatique, comprenant pleurs, essoufflement, transpiration, nausées, battement de cœur rapide, troubles fonctionnels gastro-intestinaux et tension artérielle élevée.

Le niveau d'anxiété ou de dépression chez un sujet peut être établit par le biais d'une « Échelle d'évaluation des symptômes ». Ce terme fait référence à l'un des nombreux questionnaires standardisés, instruments cliniques ou inventaires de symptômes utilisés pour mesurer les symptômes et la gravité des symptômes dans les troubles de l'état émotionnel d'un sujet.

Les échelles d'évaluation de la dépression incluent, sans toutefois s'y limiter, celles définies dans le Manuel diagnostique et statistique des troubles mentaux IV-texte révisé ( Diagnostic and Statistical Manual of Mental Disorders, DSM-IV-TR), le mini-examen de l'état mental (MMSE), l'échelle de dépression de Hamilton (HAMD-28 ou HAMD-7), l'échelle d'évaluation de la dépression de Hamilton à 17 éléments (SDRH 17), l'échelle d'évaluation clinique globale (CGI), l'inventaire rapide de l'auto-évaluation de la symptomatologie dépressive (QIDS-SR 16), l'échelle d'évaluation de la dépression de Montgomery-Asberg (MADRS), l'inventaire de dépression de Beck (BDI), l'échelle de dépression de Zung Self-Rating, l'échelle de dépression gériatrique (GDS), l'échelle d'évaluation de la dépression de Wechsler, l'échelle d'évaluation de la dépression de Raskin, l'inventaire de la symptomatologie dépressive (IDS) et l'inventaire rapide de la symptomatologie dépressive (QIDS). Par exemple, le système DSM-IV-TR pour le diagnostic du trouble dépressif majeur nécessite la présence d'au moins cinq des neuf symptômes dépressifs, notamment une humeur dépressive, une diminution de l'intérêt ou du plaisir, une perte ou un gain de poids significatif, un trouble du sommeil (par exemple, insomnie ou hypersomnie), une agitation ou un ralentissement psychomoteur, une fatigue ou perte d'énergie, un sentiment de dévalorisation ou de culpabilité excessive, une diminution de la concentration et le développement d'idées suicidaires. De préférence, les symptômes doivent être présents pendant un temps déterminé et avec une sévérité importante.

Les échelles d'évaluation de l'anxiété incluent, sans toutefois s'y limiter, l'inventaire d'anxiété caractéristique (STAI), l'échelle d'évaluation de l'anxiété de Hamilton (HAM-A), l'inventaire d'anxiété de Beck (MTD), et l'échelle d'anxiété et de dépression hospitalière (HADS-A).

Ces échelles de notation peuvent impliquer l'auto-évaluation du patient ou être notées par un clinicien. Une réduction d'au moins 50% du score de l'échelle d'évaluation de la dépression ou de l'anxiété au cours d'un essai clinique (point de départ au point final) est généralement considérée comme une réponse favorable pour la plupart des échelles d'évaluation des symptômes de la dépression et de l'anxiété.

La «rémission» dans les études cliniques sur la dépression ou l'anxiété consiste souvent à obtenir un score d'évaluation numérique particulier ou inférieur à celui-ci sur une échelle d'évaluation des symptômes de l'anxiété (par exemple, inférieur ou égal à 39 sur la STAI; ou inférieur ou égal à 9 pour le BAI; ou inférieur ou égal à 7 sur le HADS-A) ou de la dépression (par exemple, inférieur ou égal à 7 sur le SDRH 17; ou inférieur ou égal à 5 sur le QIDS-SR 16 ou inférieur ou égal à 10 sur le MADRS).

Selon un aspect, l'administration de la composition selon l'invention permet l'amélioration et/ou la réduction des scores des tests d'évaluation de l'état anxieux ou dépressif. Par exemple, un score de 0 à 7 sur l'échelle HAMD est généralement considéré comme normal. Des scores de 20 ou plus indiquent des symptômes dépressifs modérés, sévères ou très graves. Ainsi, une réduction des symptômes peut être considérée comme cliniquement pertinente si, par exemple, le score HAMD est réduit à moins de 20, par exemple.

La référence au traitement et à la prévention de la dépression, de l'anxiété ou d'un trouble dépressif ou lié à l'anxiété, telle qu'utilisée ici, inclut, entre autres, l'inhibition ou le soulagement, au moins en partie, d'un ou plusieurs symptômes du trouble anxieux et/ou du trouble de l'humeur, en particulier liés à la dépression, notamment tels que décrits ci-dessus.

Selon un mode de réalisation, l'invention concerne donc l'utilisation d'une composition pharmaceutique, nutraceutique ou alimentaire comprenant une combinaison de trois ingrédients, à savoir, de la glutamine, un extrait de curcuma et des bactéries *Lactobacillus rhamnosus,* destinée au traitement du corps humain ou animal. Cette composition est adaptée à la prévention et/ou traitement des troubles anxieux et/ou des troubles de l'humeur, qu'ils soient chroniques ou aigus. Cette composition est adaptée à la prévention et/ou au traitement de la dépression et/ou des troubles anxio-dépressifs.

La quantité thérapeutiquement efficace ou suffisante d'une composition selon l'invention, est une quantité permettant d'obtenir l'effet recherché, à savoir un effet favorisant une régulation ou une diminution des troubles anxieux et/ou des troubles de l'humeur, en particulier, une diminution des symptômes associés à la dépression et/ou à l'anxiété. Alternativement, la quantité thérapeutiquement efficace ou suffisante d'une composition pharmaceutique selon l'invention, est une quantité permettant d'améliorer le bien-être mental d'un sujet ou d'améliorer les scores des échelles d'évaluation mentale d'un sujet telles que décrites ci-dessus.

Dans une méthode de traitement d'un trouble anxieux et/ou d'un trouble de l'humeur qui n'est pas revendiquée, une "quantité thérapeutiquement efficace" d'une composition est une quantité qui réduit la sévérité d'un symptôme et/ou réduit un paramètre mesurable associé au trouble d'au moins environ 10%, au moins environ 20%, au moins environ 30%, au moins environ 40%, au moins environ 50%, au moins environ 60%, au moins environ 70%, au moins environ 80%, ou au moins environ 90% ou plus, en comparaison avec le symptôme (par exemple, la sévérité du symptôme), ou en comparaison avec le paramètre mesurable, en l'absence de traitement du sujet ou en comparaison avec un état émotionnel préalable du sujet.

Les méthodes de traitement non revendiquées impliquent généralement l'administration à un individu qui en a besoin d'une quantité efficace d'une formulation comprenant de la glutamine, un extrait de curcuma et des bactéries de l'espèce *Lactobacillus rhamnosus,* telle que décrite ci-dessus, soit dans un but curatif soit dans un but préventif afin de diminuer, limiter ou retarder l'apparition et/ou la sévérité des symptômes émotionnels. Les méthodes de traitement d'un trouble anxieux et/ou d'un trouble de l'humeur comprennent des méthodes de traitement d'individus chez lesquels on a diagnostiqué un trouble émotionnel, en particulier une anxiété et/ou une dépression ; des méthodes de réduction de l'incidence de la récurrence, ou de «poussée» du trouble; des méthodes de réduction du risque de développer des symptômes liés aux troubles émotionnels chez un individu chez qui on a diagnostiqué un trouble émotionnel et qui ont été soignés par des traitements conventionnels et sont en rémission ; et des procédés de traitement d'un trouble anxieux et/ou d'un trouble de l'humeur chez un individu qui n'a pas répondu favorablement à une thérapie conventionnelle pour traiter le trouble diagnostiqué. Les méthodes de traitement d'un trouble anxieux et/ou d'un trouble de l'humeur comprennent également des méthodes de traitement d'individus à risque. Un individu "à risque" d'un symptôme dépressif et/ou anxieux inclut des individus susceptibles de développer des symptômes anxio-dépressifs (par exemple, génétiquement prédisposé ou faisant face à un évènement tragique pouvant engendrer un tel état), un individu en rémission d'un symptôme dépressif et/ou anxieux et chez qui on diagnostique maintenant une rechute ou une prédisposition à une rechute.

En conséquence, certains aspects des procédés de traitement ou de prévention d'un symptôme dépressif ou anxieux décrit dans la présente comprennent le diagnostic de l'individu comme présentant un symptôme dépressif ou anxieux, ou risquant de développer le symptôme dépressif ou anxieux, par exemple avant de commencer l'administration de la composition divulguée. Dans certains modes de réalisation, la composition est administrée à un individu soumis à un dépistage d'un symptôme dépressif ou anxieux, par exemple, conformément à l'un des tests décrit précédemment. Le diagnostic peut notamment être posé grâce aux échelles d'évaluation de l'état émotionnel d'un sujet telles que décrites ci-dessus.

La composition selon l'invention peut également influer sur d'autres conditions, troubles ou symptômes pour lesquelles les troubles émotionnels sont associés. Par exemple, la dépression est entre autres associée à un état de fatigue et de tristesse.. Les méthodes de l'invention peuvent ainsi être appliquées en tant que mesure prophylactique pour prévenir ou réduire le risque de survenue de symptômes causés par une dépression et/ou une anxiété.

La composition selon l'invention peut être combinée avec des agents conventionnels utilisés pour le traitement de la dépression et/ou de l'anxiété, le cas échéant. Ces agents peuvent être alternativement des agents connus comme antidépresseurs ou anti-anxiolytiques, notamment des antidépresseurs imipramiques, des inhibiteurs sélectifs de la recapture de la sérotonine, de la norépinéphrine ou de la noradrénaline ou encore des inhibiteurs de la monoamine oxydase. Dans un mode de réalisation, ces agents peuvent éventuellement être administrés en une quantité subthérapeutique.

De tels agents supplémentaires peuvent être administrés séparément ou inclus dans la composition selon l'invention.

Dans certains cas, la composition selon l'invention est administrée en tant que traitement d'appoint, le sujet étant traité par une psychothérapie. Dans certains aspects qui ne sont revendiqués, la méthode de traitement avec la composition selon l'invention comprend en outre le traitement du sujet par une psychothérapie.

Tel qu'utilisé ici, une "psychothérapie" fait référence à une thérapie non pharmacologique dans laquelle le sujet est psychologiquement engagé, directement ou indirectement (par exemple par le dialogue), dans le but de rétablir un état psychologique normal ; réduire le risque de développer un trouble de l'humeur et des symptômes y étant associés ; et/ou pour soulager un trouble de l'humeur et des symptômes y étant associés.

### EXEMPLES

Les solutions ont été administrées *per os* par intubation gastrique à des souris (1 gavage par jour).

Les concentrations administrées quotidiennement *per os* par intubation gastrique sont précisées dans le tableau 1.

### Table 1

**Tableau 1. Quantité de matières premières administrées par intubation gastrique aux souris**

| Matières premières | Quantité journalière Souris (35g) |
|---|---|
| Glutamine | 24 mg |
| Extrait de curtuma | 1,2 mg |
| *Lactobacillus rhamnosus GG* | 0,120 mg |

La préparation des solutions est réalisée extemporanément et l'administration est effectuée le matin. Lors des sessions comportementales les solutions sont administrées une heure avant le début de la session. L'intubation gastrique s'effectue toujours dans une pièce différente de celle où a lieu le test comportemental.

Afin d'évaluer les résultats obtenus avec les solutions, un groupe de souris a reçu une administration parentérale d'un antidépresseur tricyclique (clomipramine). Les souris ont été réparties par groupes indépendants selon les traitements suivants :

### Table 2

**Tableau 2. Répartition des animaux (n=12 par groupe)**

| Groupes (n=12 par groupe) | TRAITEMENTS |
|---|---|
| Contrôle | / |
| Contrôle | Placebo |
| Modèle induit | Placebo |
| Modèle induit | Glutamine |
| Modèle induit | *Lactobacillus rhamnosus GG* |
| Modèle induit | Extrait de curcuma |
| Modèle induit | Extrait de curcuma + Glutamine |
| Modèle induit | *Lactobacillus rhamnasus GG* + Glutamine |
| Modèle induit | Extrait de curcuma + *Lactobacillus rhaninosus GG* |
| Modèle induit | Extrait de curcuma + *Lactobacillus rhamnosus GG* + Glutamine |
| Modèle induit | Clomipramine (10mg/kg) par injection intrapéritonéale |

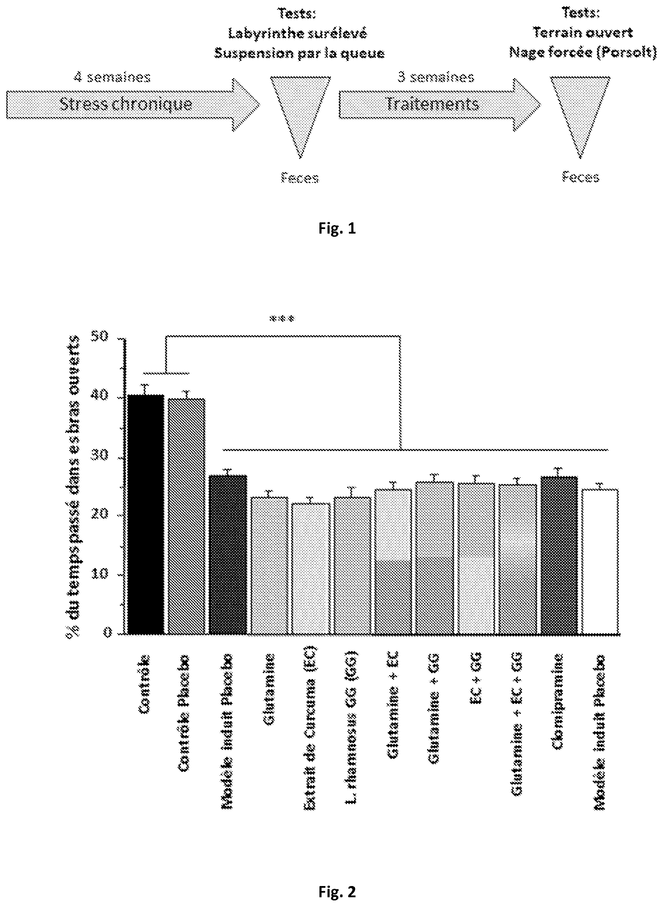

Pour chaque groupe, l'administration des différentes solutions dure 21 jours (3 semaines) consécutifs. Les tests comportementaux sont réalisés 4 semaines après l'induction du modèle (« Labyrinthe en croix surélevé » et « Suspension par la queue ») et 3 semaines après les intubations quotidiennes (« Terrain ouvert » et « Nage forcée »). Le déroulement de l'étude est présenté Figure 1.

### Les tests

Le test Labyrinthe en croix surélevé (en anglais « elevated plus maze » ou EPM)) est utilisé classiquement pour évaluer la réactivité de type anxiété chez les rongeurs (Can A, et al. J Vis Exp 2012;59:e3769). L'appareillage surélevé mesure 60 cm de haut et se compose de deux bras ouverts opposés (30 cm de long, 7 cm de large) et de deux bras fermés perpendiculaires opposés (fermés avec un mur de 17 cm de haut). Le labyrinthe est placé dans une pièce avec un éclairage de 70 lux. Au début de la session, des souris ont été placées au centre du plus-maze dans un cylindre en PVC pendant 20 secondes pour permettre une orientation aléatoire au début de l'exploration. Ensuite, après un délai de 10 secondes dans le cylindre, les souris ont été autorisées à explorer librement le labyrinthe pendant 8 minutes. Au cours de chaque séance, le temps passé dans les bras ouverts a été mesuré et le pourcentage du temps passé dans les bras ouverts a été calculé selon la formule suivante (Rapport temps : temps dans les bras ouverts / temps dans tous les bras) x100). Tous les paramètres ont été automatiquement notés par vidéotracking (ViewPoint^{®}, France).

La suspension par la queue (en anglais « tail suspension test » ou TST) est un test couramment utilisé pour évaluer le comportement dépressif chez la souris (Steru L, et al. Psychopharmacology (Berl) 1985;85(3):367-70). Comme dans le test de nage forcée, ce test est également basé sur le désespoir comportemental. Ce test consiste à fixer l'animal par la queue avec du ruban adhésif (tête en bas : 35 cm du sol) pendant 5 minutes. Au cours de cette séance, l'immobilité (sec) et le pourcentage du temps d'immobilisation, qui est un indice de comportement dépressif chez le rongeur, ont été mesurés.

Le test en terrain ouvert (en anglais « open field test») est une arène circulaire (Ø 1 m) entourée d'un mur opaque de 25 cm de haut placé dans une pièce avec un éclairage uniforme de 70 lux. Les souris sont placées face au mur à la périphérie de l'arène et peuvent l'explorer librement pendant 5 minutes. La surface du champ ouvert est pratiquement divisée en une zone périphérique (12 cm de large) et une zone centrale (la surface restante de l'arène). Le temps passé (en secondes) dans la zone centrale est automatiquement noté par vidéotracking. Plus un animal passe du temps dans la zone centrale, moins la souris est anxieuse.

Le test de nage forcée (Porsolt) est un test couramment utilisé pour évaluer le comportement dépressif chez la souris. Ce test est basé sur le désespoir comportemental. Ce test consiste à déposer l'animal dans un bassin cylindrique en verre (Ø 15cm) rempli d'eau (30 cm de haut, 25 ± 1°C) pendant 6 minutes pendant lesquelles l'animal peut nager, monter ou rester immobile. Un animal présentant un comportement dépressif passera plus de temps d'immobilité que la souris témoin non dépressive. Après la séance, l'animal est séché et placé sous une lumière chaude. Trois paramètres permettant d'évaluer le comportement dépressif sont notés : le temps passé à nager, à grimper et le temps d'immobilité de chaque animal ainsi que leur pourcentage respectif.

### RESULTATS

### Validation du modèle d'anxiété et de dépression

Après 4 semaines d'induction de l'anxiété par des stress imprévisibles, les inventeurs ont évalué la réactivité des souris à ces tests d'anxiété induite (UMCS) par rapport à deux groupes témoins appariés selon l'âge : des souris non stressées qui ne seront pas traitées, des souris non stressées qui seront soumises à une intubation gastrique placebo.

### Anxiété

L'anxiété est mesurée en réalisant un test « Labyrinthe en croix surélevé » tel que définit plus haut. Dans la figure 2, les données sont présentées en fonction de l'attribution ultérieure des différents groupes de la phase 2, même si aucun traitement n'a été administré au cours de la phase 1 actuelle. Il est mis en évidence que le temps passé dans les bras ouverts du système est significativement diminué chez les animaux stressés vs les animaux non stressés (p<0,001).

### Table 3

**Tableau 3 : Résultats du test « Labyrinthe surélevé ». Les résultats sont les moyennes +/- l'erreur-type. SEM = Moyenne Erreur Standard (Standard Error Mean)**

| **Labyrinthe en croix surélevé** | | | | |
|---|---|---|---|---|
| Groupe (n=12) | **Temps dans bras ouvert (sec)** | ***SEM*** | **Temps dans bras fermé (sec)** | ***SEM*** |
| Contrôle | 194,2 | *8,4* | 285,8 | *8,4* |
| Contrôle + Placebo | 191,6 | *6,0* | 288,4 | *6,0* |
| Modèle + Placebo | 128,5 | *4,9* | 351,5 | *4,9* |
| Modèle + *L. rhamnosus GG* | 111,3 | *8,8* | 368,8 | *8,8* |
| Modèle + Extrait de curcuma | 106,4 | *4,9* | 373,6 | *4,9* |
| Modèle + Glutamine | 111,3 | *5,3* | 368,8 | *5,3* |
| Modèle + Extrait de curcuma + Glutamine | 117,9 | *6,1* | 362,1 | *6,1* |
| Modèle + *Lactobacillus rhamnosus GG* + Glutamine | 123,9 | *5,7* | 356,1 | *5,7* |
| Modèle + Extrait de curcuma + *Lactobacillus rhamnosus GG* | 122,3 | *6,4* | 357,8 | *6,4* |
| Modèle + Extrait de curcuma + *Lactobacillus rhamnosus GG* + Glutamine | 121,8 | *4,3* | 358,3 | *4,3* |
| Modèle + Clomipramine (10mg/kg) par injection intrapéritonéale | 127,4 | *7,1* | 352,6 | *7,1* |

### Dépression

Vingt-quatre heures après le test « Labyrinthe surélevé », le comportement dépressif est évalué par le test de suspension par la queue.

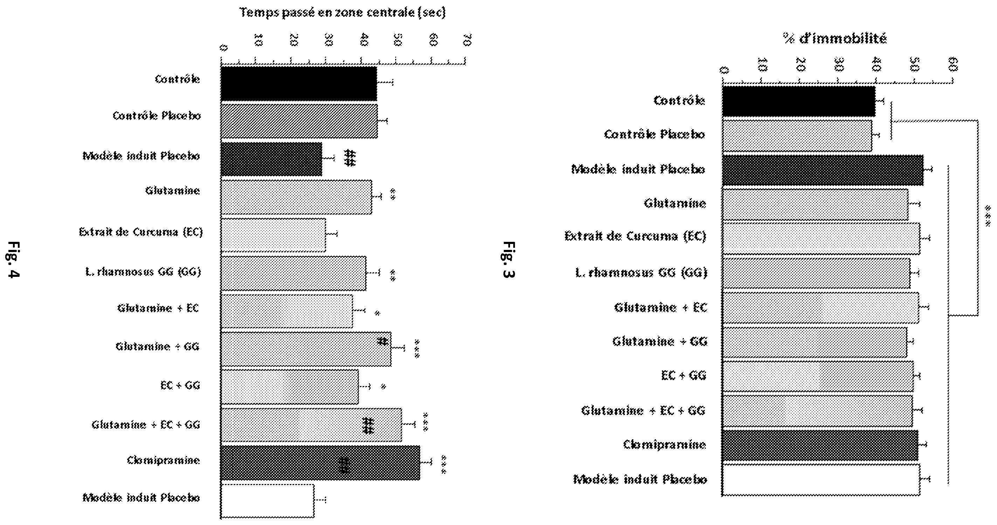

La figure 3 décrit le pourcentage de temps d'immobilité des animaux contrôles et des animaux stressés.

### Table 4

**Tableau 4 : Résultats du test de suspension par la queue. Les résultats sont les moyennes +/-l'erreur-type. SEM = Moyenne Erreur Standard (Standard Error Mean)**

| **Test de suspension par la queue** | | |
|---|---|---|
| Groupe (n=12) | Moyenne (sec) | ***SEM*** |
| Contrôle | 118,8 | *7,5* |
| Contrôle + Placebo | 116,8 | *6,2* |
| Modèle + Placebo | 157,3 | *6,4* |
| Modèle + *L. rhamnosus GG* | 146,8 | *6,9* |
| Modèle + Extrait de curcuma | 154,8 | *7,6* |
| Modèle + Glutamine | 145,5 | *9,4* |
| Modèle + Extrait de curcuma + Glutamine | 154,2 | *7,1* |
| Modèle + *Lactobacillus rhamnosus GG* + Glutamine | 143,9 | *5,3* |
| Modèle + Extrait de curcuma + *Lactobacillus rhamnosus GG* | 149,1 | *5,6* |
| Modèle + Extrait de curcuma+ *Lactobacillus rhamnosus GG* + Glutamine | 148,1 | *8,1* |
| Modèle + Clomipramine (10mg/kg) par injection intrapéritonéale | 154,4 | *8,0* |

Il est mis en évidence que les animaux stressés passent significativement plus de temps immobile comparés aux animaux contrôles (p<0,001).

### Conclusion

L'ensemble de ces résultats indiquent que 4 semaines d'exposition à des stress imprévisibles (UMCS) ont provoqué un fort état d'anxiété et de dépression chez des souris âgées de 6 mois. Plus important encore, tous les groupes évalués au cours de la phase 2 présentaient des niveaux similaires d'anxiété et de comportement dépressif avant l'administration des ingrédients seuls ou en combinaison.

### Evaluation de la formulation et de ses ingrédients seuls ou en combinaison

### Anxiété

Le comportement anxieux a été évalué avec le test en terrain ouvert. Il est mesuré le temps moyen passé par les souris au centre de l'appareil.

La figure 4 présente les effets des différentes associations en comparaison des groupes témoins non stressés et du groupe « modèle d'anxiété et placebo », dit groupe Modèle induit Placebo.

### Table 5

**Tableau 5. Résultats du test « Terrain ouvert »**

| **Test Terrain ouvert** | | |
|---|---|---|
| Groupe (n=12) | Moyenne (sec) | *SEM* |
| Contrôle | 44,3 | *5* |
| Contrôle + Placebo | 44,7 | *2,8* |
| Modèle + Placebo | 28,8 | *3,7* |
| Modèle + *L. rhamnosus GG* | 41,6 | *3,6* |
| Modèle + Extrait de curcuma | 29,8 | *3,3* |
| Modèle + Glutamine | 42,9 | *3* |
| Modèle + Extrait de curcuma + Glutamine | 37,7 | *3,4* |
| Modèle + *Lactobacillus rhamnosus GG* + Glutamine | 48,5 | *3,8* |
| Modèle + Extrait de curcuma + *Lactobacillus rhamnosus GG* | 39,3 | *3,5* |
| Modèle + Extrait de curcuma + *Lactobacillus rhamnosus GG* + Glutamine | 51,7 | *3,6* |
| Modèle + Clomipramine (10mg/kg) par injection intrapéritonéale | 56,8 | *3,4* |

### Dépression

### Temps d'immobilité (Figure 5A et tableau 6) :

L'analyse statistique en ANOVA unidirectionnelle met en évidence une différence significative entre les groupes, les différents traitements n'induisant pas les mêmes effets sur les symptômes de la dépression et de l'anxiété (F(11,132) = 20.735 ; *p* < 0.001).

En comparaison au "groupe Modèle + Placebo", les analyses *post hoc* mettent en évidence que tous les traitements exceptés le curcumin (*p* = 0.14) diminue le temps d'immobilité jusqu'à normaliser son niveau à celui des animaux non stressés (*p*<0,001 pour tous). De plus, il apparait que le traitement Glutamine + Curcuma + *Lactobacillus rhamnosus GG* est significativement plus efficace que ces trois ingrédients testés seuls ou en association deux à deux (p<0,001 pour tous les groupes) et comparable au traitement médicamenteux par injection intrapéritonéale (p=0,13). Enfin le groupes « Glutamine + Curcuma + *Lactobacillus rhamnosus GG* » diminue le temps d'immobilité de façon significative comparé aux animaux non stressés et non traités (p<0,001) et de façon comparable au groupe « Clomipramine ».

### Temps d'Ascension (Climbing) (Figure 5B et tableau 6) :

L'analyse statistique en ANOVA unidirectionnelle met en évidence une différence significative entre les groupes (F(11,132) = 26.504; *p* < 0.001). En comparaison au "groupe Modèle + Placebo", les analyses *post hoc* mettent en évidence que tous les traitements exceptés le curcumin (*p* = 0.25) augmentent le temps passé à grimper chez les souris stressées.

De plus, il apparait que le traitement Glutamine + Curcuma *+ Lactobacillus rhamnosus GG* est significativement plus efficace que ces trois ingrédients testés seuls ou en association deux à deux (p<0,001 pour tous les groupes) et comparable au traitement médicamenteux par injection intrapéritonéale (p=0,11).

### Temps de nage (Figure 5C et tableau 6) :

Pour finir, l'analyse statistique en ANOVA unidirectionnelle met en évidence une différence significative entre les groupes (F(11,132) = 1.922; p = 0.004), les groupes « Glutamine + Curcuma *+ Lactobacillus rhamnosus GG »* et «clomipramine » passant plus de temps à nager comparé aux autres groupes.

### Composition du microbiote par analyse de l'ARN16S

La composition du microbiote des animaux a été analysée afin de déterminer l'influence de la formule sur la composition bactérienne du microbiote fécal des souris stressées et de la comparer avec l'influence du médicament ou du placebo.

Les fèces des animaux ont été échantillonnées afin qu'une extraction d'ADN soit réalisée (Zymo Research based kits). Les régions V3-V4 du gène ARN16S sont amplifiées et le séquençage est réalisé sur système ILLUMINA MiSeq utilisant le mode 2*250 bp. L'analyse des séquences est réalisée après correction en utilisant SPAdes (Bak et al, J Neurochem 2006;98(3):641-53) et le regroupement en utillisant PEAR (Zhang J, et al. Bioinformatics 2014;30(5):614-20). Les unités taxonomiques opérationnelles ou OTUs (Operational Taxonomic Units) sont identifiées avec l'outil Vsearch (Rognes T, et al. Peer J 2016;4:e2584), la classification taxonomique en découlant a été effectuée à l'aide de la base de données RDP (Ribosomal Database Project).

La majorité des études de diversité basées sur l'ADNr 16S définissent les OTUs comme étant des ensembles de séquences présentant au moins 97% d'identité entre elles. Les OTUs sont en fait l'unité de mesure de la richesse spécifique en écologie microbienne.

### Statistiques

Les analyses statistiques ont été effectuées en utilisant le programme R (Team RDC. R: A Language and Environment for Statistical Computing. 2012, Austria: R Foundation for Statistical Computing) et différents paquets (Ade4, Vegan). Les post-tests de Wilcoxon ou de Kruskal-Wallis ont été utilisés pour comparer les groupes. Les tests appariés de Wilcoxon ont été utilisés pour les comparaisons par paires entre les groupes, avec des corrections pour les tests multiples. Plusieurs tests ont été corrigés à l'aide de la méthode du taux de fausses découvertes (valeur q).

### Analyse et résultats

Un total de 3 824 OTUs a été obtenu avec une moyenne de 1 312 ± 159 OTUs détectées par échantillon.

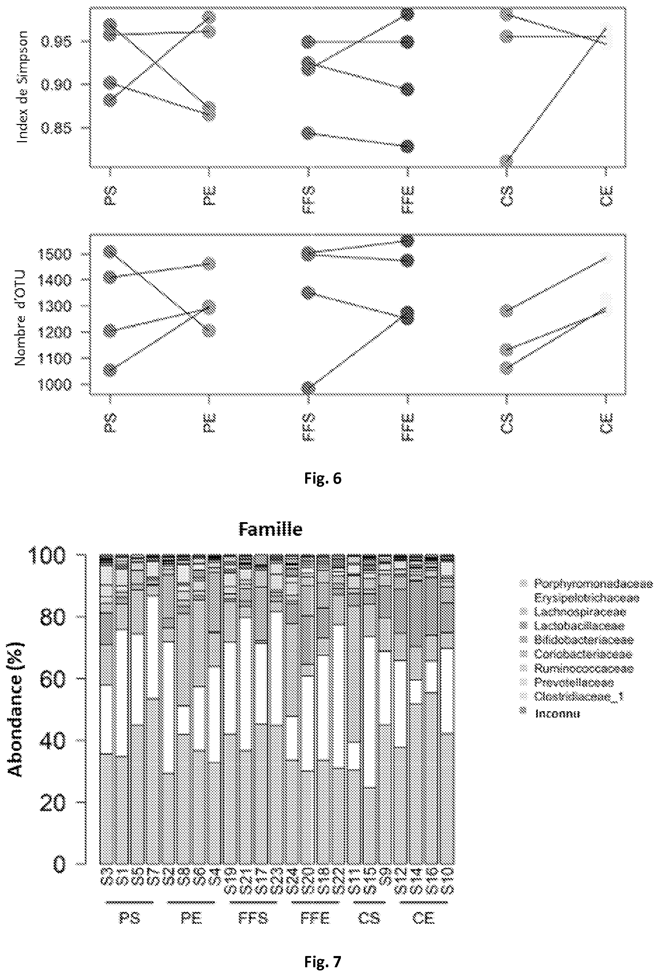

Aucune différence significative n'a été mise en évidence entre les groupes avant ou après traitement. La diversité microbienne (α-diversité) est quasi-identique d'un échantillon à l'autre (Figure 6)

La composition du microbiote a montré une répartition similaire des familles bactériennes entre les échantillons, les Porphyromonadaceae (39±8%) et les Erysipelotrichaceae (28±12%) étant les familles bactériennes les plus abondantes (Figure 7).

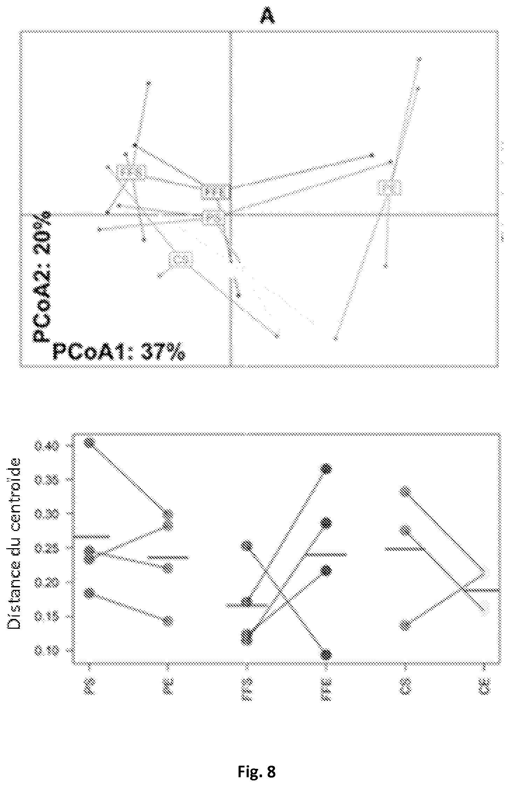

Comme l'indique la Figure 8 - A et selon l'axe PCoA1, le groupe placebo présente le profil microbiotal qui a le plus évolué par rapport aux autres groupes (formule complète et clomipramine). Cela signifie que les traitements (formule complète et clomipramine). stabiliseraient la dysbiose induite par les 4 semaines de stress par rapport au groupe placebo dont l'écosystème intestinal continue à dériver.

En conclusion, cette étude a montré que 4 semaines d'UMCS ont induit de l'anxiété et des symptômes dépressifs chez des souris âgées de 6 mois, en comparaison avec des souris non stressées. De plus, les souris stressées présentent toujours une augmentation de l'anxiété et des comportements dépressifs 28 jours après l'arrêt de la procédure UMCS, ce qui indique une augmentation sévère et résistante à long terme de la réactivité à la peur. Nous avons démontré que tous les traitements chroniques, à l'exception de l'extrait de curcuma administré seul, ont diminué l'anxiété et les symptômes dépressifs.

De plus, il a été observé que le traitement chronique avec l'association glutamine + curcuma + *Lactobacillus rhamnosus GG* est la combinaison la plus efficace permettant i) la restauration d'un niveau normal de réactivité émotionnelle, aussi puissante que la clomipramine, par rapport au groupe stressé + placebo, ii) une réduction significative de la réactivité à la peur et du comportement dépressif par rapport aux témoins non stressés, iii) une modulation de la composition microbiotale par rapport au groupe placebo (stabilisation d'une dysbiose induite par le stress).

Ainsi, l'association de glutamine + curcuma *+ Lactobacillus rhamnosus GG* a induit un effet microbiotal associé à un effet anxiolytique et antidépresseur. Ces effets sont comparables à ceux obtenus avec la clomipramine.

La combinaison selon l'invention permet donc après 21 jours de traitement une potentialisation de l'effet par rapport aux ingrédients seuls ou combinés deux à deux. Cette combinaison permet après 21 jours de traitement d'obtenir un effet comparable à celui de la clomipramine administrée en intra-péritonéal.

### Table 6

**Tableau 6 : Résultats du test de nage forcée. A - Pourcentage du temps d'immobilité; B - Pourcentage du temps d'escalade; C - Pourcentage du temps de nage. Les résultats sont les moyennes +/- l'erreur-type.**

| Groupe (n=12) | **Immobilité (sec)** | ***SEM*** | **Escalade (sec)** | ***SEM*** | **Nage (sec)** | ***SEM*** |
|---|---|---|---|---|---|---|
| Contrôle | 100,3* | *6,7* | 122,0 | *4,2* | 137,7 | *4,9* |
| Contrôle + Placebo | 96,3* | *7,3* | 121,9 | *2,8* | 141,8 | *6,8* |
| Modèle + Placebo | 143,4* | *4,9* | 79,3* | *3,8* | 137,3 | *6,4* |
| Modèle + *Lactobacillus rhamnosus GG* | 106,8* | *5,0* | 113,5* | *2,5* | 139,8 | *6,9* |
| Modèle + Extrait de curcuma | 132,8* | *5,8* | 85,1* | *4,6* | 142,2 | *6,5* |
| Modèle + Glutamine | 111,0* | *2,9* | 107,3* | *3,9* | 141,8 | *3,6* |
| Modèle + Extrait de curcuma + Glutamine | 119,4* | *2,4* | 107,3* | *3,7* | 133,3 | *3,7* |
| Modèle + *Lactobacillus rhamnosus GG* + Glutamine | 98,5* | *5,6* | 119,8* | *3,8* | 141,8 | *6,5* |
| Modèle + Extrait de curcuma + *Lactobacillus rhamnosus GG* | 119,3* | *3,3* | 109,9* | *2,3* | 130,8 | *3,2* |
| Modèle + Extrait de curcuma + *Lactobacillus rhamnosus GG* + Glutamine | 74,5 | *4,5* | 130,3 | *4,2* | 155,3 | *5,6* |
| Modèle + Clomipramine (10mg/kg) par injection intrapéritonéale | 63,6 | *5,3* | 138,3 | *3,8* | 158,1 | *6,6* |

### Exemples de formulation de la composition

**Table 7**

| | Stick-packs | |
|---|---|---|
| | mg | % |
| Glutamine | 2000 | 90,29 |
| Extrait de curcuma | 100 | 4,51 |
| *Lactobacillus rhamnosus GG* | 10 | 0,45 |
| Maltodextrine | 100 | 4,51 |
| Dioxyde de silice | 5 | 0,22 |
| TOTAL | 2215 | 100 |

### Exemple 1

| | mg pour une gélule | % |
|---|---|---|
| Glutamine | 500 | 76,92 |
| Extrait de curcuma | 50 | 7,69 |
| *Lactobacillus rhamnosus GG* | 20 | 3,07 |
| Maltodextrine | 75 | 11,53 |
| Stéarate de magnésium | 5 | 0,76 |
| TOTAL | 650 | 100 |

### Exemple 2

| | mg pour deux gélules | % |
|---|---|---|
| Glutamine | 1000 | 86,02 |
| Extrait de curcuma | 50 | 4,3 |
| *Lactobacillus rhamnosus GG* | 10 | 0,86 |
| Maltodextrine | 100 | 8,6 |
| Stéarate de magnésium | 2,5 | 0,21 |
| TOTAL | 1162,5 | 100 |

Exemple 3

## Revendications

1. Composition pharmaceutique, nutraceutique ou alimentaire comprenant de la glutamine, un extrait de curcuma et du *Lactobacillus rhamnosus,* **caractérisée en ce que** la glutamine, l'extrait de curcuma et le *Lactobacillus rhamnosus* sont présents dans la composition dans les proportions suivantes :
- de 90 à 96% en poids de glutamine,
- de 3 à 6% en poids d'extrait de curcuma,
- de 0,25% à 5% en poids de *Lactobacillus rhamnosus,*
pour 100% en poids de la somme de glutamine, d'extrait de curcuma et *Lactobacillus rhamnosus,* et dans laquelle l'extrait de curcuma comprend au moins 15% en poids de curcuminoïdes et est associé à une cyclodextrine.

2. Composition selon la revendication 1, **caractérisée en ce que** la composition comprend de 90 à 96% en poids de glutamine, de 3 à 6% en poids d'extrait de curcuma, et de 0,25 à 5% en poids de *Lactobacillus rhamnosus,* pour 100% en poids de la composition pharmaceutique, nutraceutique ou alimentaire.

3. Composition selon la revendication 1 ou 2, dans laquelle la cyclodextrine est une gamma-cyclodextrine.

4. Composition selon l'une quelconque des revendications 1 à 3, dans laquelle la souche de *Lactobacillus rhamnosus* est la souche *Lactobacillus rhamnosus GG.*

5. Composition selon l'une quelconque des revendications 1 à 4, comprenant en outre :
- un ou plusieurs prébiotiques ; et/ou
- des vitamines, minéraux et/ou oligo-élements ; et/ou
- des poudres ou extraits de plantes, fruits, légumes, algues ou champignons ; et/ou
- un ou plusieurs probiotiques choisis dans le groupe constitué par des bactéries du genre Bacillus, Bacteroides, Enterobacteriaceae, Enterococcus, Faecalibacterium, Fusobacterium, Lactobacillus, Odoribacter, Parabacteroides, Pediococcus, Roseburia, Ruminococcus, et Streptococcus et/ou des levures du genre Saccharomyces.

6. Composition selon une quelconque des revendications 1 à 5, pour son utilisation en tant que complément alimentaire ou médicament, en particulier à usage humain ou vétérinaire.

7. Composition selon l'une quelconque des revendications 1 à 5, pour son utilisation dans la prévention ou le traitement de la dépression ou de l'anxiété et dans laquelle la composition est administrée par voie orale.

8. Composition pour une utilisation selon l'une quelconque des revendications 6 à 7, **caractérisée en ce que** la composition est dans une forme adaptée pour une administration journalière de glutamine entre 0,5 et 10 g, d'extrait de curcuma entre 25 mg et 500 mg et de *Lactobacillus rhamnosus* entre 0,05 mg et 500 mg ou entre 1x10⁷ CFU et 1x10¹¹ CFU.

9. Composition selon l'une quelconque des revendications 1 à 5, ladite composition se présentant sous la forme d'unités de dose, par exemple sous la forme de poudres, de comprimés ou de gélules, chaque unité de dose comprenant :
- de 0,5 g à 10 g de glutamine, de préférence de 0,5 g à 2 g de glutamine,
- de 25 mg à 500 mg d'extrait de curcuma, de préférence de 50 mg à 100 mg d'extrait de curcuma, et
- de 0,05 mg à 500 mg ou entre 1x10⁷ CFU et 1x10¹¹ CFU de *Lactobacillus rhamnosus,* de préférence de 10 mg à 20 mg de *Lactobacillus rhamnosus.*

10. Kit pharmaceutique, nutraceutique ou alimentaire comprenant la composition selon l'une quelconque des revendication 1 à 5 et 8 à 9, dans lequel la composition est sous la forme de compositions distinctes, comprenant :
- une première composition comprenant la bactérie *Lactobacillus rhamnosus,* par exemple associée à un ou plusieurs excipients, et
- une seconde composition comprenant la glutamine et l'extrait de curcuma, par exemple associée à un ou plusieurs excipients.

11. Kit pharmaceutique, nutraceutique ou alimentaire selon la revendication 10, pour son utilisation en tant que complément alimentaire ou médicament, en particulier à usage humain ou vétérinaire.

12. Kit pharmaceutique, nutraceutique ou alimentaire pour son utilisation selon la revendication 10, pour son utilisation dans la prévention ou le traitement de la dépression ou de l'anxiété.

## Patentansprüche

1. Pharmazeutische, nutrazeutische oder Lebensmittelzusammensetzung, umfassend Glutamin, einen Kurkumaextrakt und *Lactobacillus rhamnosus,* **dadurch gekennzeichnet, dass** Glutamin, Kurkumaextrakt und *Lactobacillus rhamnosus* in der Zusammensetzung in den folgenden Anteilen vorhanden sind:
- zu 90 bis 96 Gew.-% Glutamin,
- zu 3 bis 6 Gew.-% Kurkumaextrakt,
- zu 0,25 Gew.-% bis 5 Gew.-% *Lactobacillus rhamnosus,*
für 100 Gew.-% der Summe aus Glutamin, Kurkumaextrakt und *Lactobacillus rhamnosus,* und wobei der Kurkumaextrakt mindestens 15 Gew.-% Curcuminoide umfasst und mit einem Cyclodextrin verbunden ist.

2. Zusammensetzung nach Anspruch 1, **dadurch gekennzeichnet, dass** die Zusammensetzung zu 90 bis 96 Gew.-% Glutamin, zu 3 bis 6 Gew.-% Kurkumaextrakt und zu 0,25 bis 5 Gew.-% *Lactobacillus rhamnosus* umfasst, für 100 Gew.-% der pharmazeutischen, nutrazeutischen oder Lebensmittelzusammensetzung.

3. Zusammensetzung nach Anspruch 1 oder 2, wobei das Cyclodextrin ein Gamma-Cyclodextrin ist.

4. Zusammensetzung nach einem der Ansprüche 1 bis 3, wobei der Stamm *von Lactobacillus rhamnosus* der Stamm *Lactobacillus rhamnosus GG* ist.

5. Zusammensetzung nach einem der Ansprüche 1 bis 4, ferner umfassend:
- ein oder mehrere Präbiotika; und/oder
- Vitamine, Mineralstoffe und/oder Spurenelemente; und/oder
- Pulver oder Extrakte aus Pflanzen, Früchten, Gemüse, Algen oder Pilzen; und/oder
- ein oder mehrere Probiotika, die aus der Gruppe ausgewählt sind, bestehend aus Bakterien der Gattung Bacillus, Bacteroides, Enterobacteriaceae, Enterococcus, Faecalibacterium, Fusobacterium, Lactobacillus, Odoribacter, Parabacteroides, Pediococcus, Roseburia, Ruminococcus und Streptococcus und/oder Hefen der Gattung Saccharomyces.

6. Zusammensetzung nach einem der Ansprüche 1 bis 5 zur Verwendung als Nahrungsergänzungsmittel oder Arzneimittel, insbesondere zur Verwendung bei Menschen oder Tieren.

7. Zusammensetzung nach einem der Ansprüche 1 bis 5 zur Verwendung bei der Vorbeugung oder der Behandlung von Depressionen oder Angstzuständen und wobei die Zusammensetzung oral verabreicht wird.

8. Zusammensetzung zur Verwendung nach einem der Ansprüche 6 bis 7, **dadurch gekennzeichnet, dass** die Zusammensetzung in einer Form vorliegt, die für eine tägliche Verabreichung von Glutamin zwischen 0,5 und 10 g, Kurkumaextrakt zwischen 25 mg und 500 mg und *Lactobacillus rhamnosus* zwischen 0,05 mg und 500 mg oder zwischen 1x10⁷ KBE und 1x10¹¹ KBE angepasst ist.

9. Zusammensetzung nach einem der Ansprüche 1 bis 5, wobei die Zusammensetzung in Form von Dosiseinheiten vorliegt, beispielsweise in Form von Pulvern, Tabletten oder Kapseln, jede Dosiseinheit umfassend:
- 0,5 g bis 10 g Glutamin, vorzugsweise 0,5 g bis 2 g Glutamin,
- 25 mg bis 500 mg Kurkumaextrakt, vorzugsweise 50 mg bis 100 mg Kurkumaextrakt, und
- von 0,05 mg bis 500 mg oder zwischen 1x10⁷ KBE und 1x10¹¹ KBE von *Lactobacillus rhamnosus,* vorzugsweise 10 mg bis 20 mg *Lactobacillus rhamnosus.*

10. Pharmazeutisches, nutrazeutisches oder Nahrungsmittelkit, umfassend die Zusammensetzung nach einem der Ansprüche 1 bis 5 und 8 bis 9, wobei die Zusammensetzung in Form separater Zusammensetzungen ist, umfassend:
- eine erste Zusammensetzung, umfassend das Bakterium *Lactobacillus rhamnosus,* beispielsweise in Verbindung mit einem oder mehreren Hilfsstoffen, und
- eine zweite Zusammensetzung, umfassend Glutamin und Kurkumaextrakt, beispielsweise in Verbindung mit einem oder mehreren Hilfsstoffen.

11. Pharmazeutisches, nutrazeutisches oder Nahrungsmittelkit nach Anspruch 10 zur Verwendung als Nahrungsergänzungsmittel oder Arzneimittel, insbesondere zur Verwendung bei Menschen oder Tieren.

12. Pharmazeutisches, nutrazeutisches oder Nahrungsmittelkit zur Verwendung nach Anspruch 10 zur Verwendung bei der Vorbeugung oder der Behandlung von Depressionen oder Angstzuständen.

## Claims

1. Pharmaceutical, nutraceutical or food composition comprising glutamine, turmeric extract and *Lactobacillus rhamnosus,* **characterized in that** glutamine, turmeric extract and *Lactobacillus rhamnosus* are present in the composition in the following proportions:
- 90 to 96% by weight of glutamine,
- 3 to 6% by weight of turmeric extract,
- 0.25% to 5% by weight of *Lactobacillus rhamnosus,*
for 100% by weight of the sum of glutamine, turmeric extract and *Lactobacillus rhamnosus,* and the turmeric extract comprising at least 15% by weight of curcuminoids and being combined with a cyclodextrin.

2. Composition according to claim 1, **characterized in that** the composition comprises 90 to 96% by weight of glutamine, 3 to 6% by weight of turmeric extract, and 0.25 to 5% by weight of *Lactobacillus rhamnosus,* for 100% by weight of the pharmaceutical, nutraceutical or food composition.

3. Composition according to claim 1 or 2, wherein the cyclodextrin is a gamma-cyclodextrin.

4. Composition according to any of claims 1 to 3, wherein the *Lactobacillus rhamnosus* strain is *Lactobacillus rhamnosus GG*.

5. Composition according to any of claims 1 to 4, further comprising:
- one or more prebiotics; and/or
- vitamins, minerals and/or trace elements; and/or
- powders or extracts of plants, fruits, vegetables, algae or mushrooms; and/or
- one or more probiotics selected from the group consisting of bacteria of the genus Bacillus, Bacteroides, Enterobacteriaceae, Enterococcus, Faecalibacterium, Fusobacterium, Lactobacillus, Odoribacter, Parabacteroides, Pediococcus, Roseburia, Ruminococcus, and Streptococcus and/or yeasts of the genus Saccharomyces.

6. Composition according to any of claims 1 to 5, for use as a food supplement or medicament, in particular for human or veterinary use.

7. Composition according to any of claims 1 to 5, for use in the prevention or treatment of depression or anxiety and wherein the composition is administered orally.

8. Composition for use according to any of claims 6 to 7,
**characterized in that** the composition is in a form suitable for daily administration of glutamine between 0.5 and 10 g, turmeric extract between 25 mg and 500 mg and *Lactobacillus rhamnosus* between 0.05 mg and 500 mg or between 1x10⁷ CFU and 1x10¹¹ CFU.

9. Composition according to any of claims 1 to 5, said composition being in the form of unit doses, for example in the form of powders, tablets or capsules, each unit dose comprising:
- 0.5 g to 10 g of glutamine, preferably 0.5 g to 2 g of glutamine,
- 25 mg to 500 mg of turmeric extract, preferably 50 mg to 100 mg of turmeric extract, and
- 0.05 mg to 500 mg or between 1x10⁷ CFU and 1x10¹¹ CFU of *Lactobacillus rhamnosus,* preferably 10 mg to 20 mg of *Lactobacillus rhamnosus.*

10. Pharmaceutical, nutraceutical or food kit comprising the composition according to any of claims 1 to 5 and 8 to 9, wherein the composition is in the form of separate compositions, comprising:
- a first composition comprising the bacterium *Lactobacillus rhamnosus,* for example combined with one or more excipients, and
- a second composition comprising glutamine and turmeric extract, for example combined with one or more excipients.

11. Pharmaceutical, nutraceutical or food kit according to claim 10, for use as a food supplement or medicament, in particular for human or veterinary use.

12. Pharmaceutical, nutraceutical or food kit for use according to claim 10, for use in the prevention or treatment of depression or anxiety.
